# EUROPEAN PATENT SPECIFICATION

(11) **EP 1 239 885 B1**
(45) Date of publication and mention of the grant of the patent: **11.06.2008**
(21) Application number: 00988400.8
(22) Date of filing: 22.12.2000
(51) Int. Cl.: A61K 49/00, C07H 21/04

(54) **INTERVENTIONS TO MIMIC THE EFFECTS OF CALORIE RESTRICTION**
MASSNAHMEN WELCHE DIE EFFEKTE EINER KALORISCHEN BESCHRÄNKUNG NACHAHMEN
INTERVENTIONS IMITANT LES EFFETS D'UNE RESTRICTION CALORIQUE

(30) Priority: 23.12.1999 US 471224; 25.08.2000 US 648642
(43) Date of publication of application: 18.09.2002
(73) Proprietor: The Regents of the University of California, Oakland, CA 94607-5200 (US)
(72) Inventor: SPINDLER, Stephen, R., Riverside, CA 92506 (US); DHAHBI, Joseph, M., Alameda, CA 94501 (US)
(74) Representative: Campbell, Patrick John Henry
(86) International application number: PCT/US2000/035437
(87) International publication number: WO 2001/045752

(56) References cited:
- WO-A-01/12851
- DATABASE MEDLINE [Online] November 1999 (1999-11) TURTURRO A ET AL: "Growth curves and survival characteristics of the animals used in the Biomarkers of Aging Program." Database accession no. NLM10619312 XP002239886 & THE JOURNALS OF GERONTOLOGY. SERIES A, BIOLOGICAL SCIENCES AND MEDICAL SCIENCES. UNITED STATES NOV 1999, vol. 54, no. 11, November 1999 (1999-11), pages B492-B501, ISSN: 1079-5006
- MOTE P.L. ET AL.: 'Glucose regulation of GRP78 gene expression' MECHANISMS OF AGING AND DEVELOPMENT vol. 104, no. 2, August 1998, pages 149 - 158, XP002938169
- WACHSMAN J.T.: 'The benificial effects of dietary restriction: reduced oxidative damage and enhanced apoptosis' MUTATION RESEARCH vol. 350, no. 1, 1996, pages 25 - 34, XP002938170
- GILL R.T. ET AL.: 'Genomic analysis of high-cell density recombinant escherichia coli fermentation and 'cell conditioning' for improved recombinant protein yield' BIOTECHNOLOGY AND BIOENGINEERING vol. 72, no. 1, 05 January 2001, pages 85 - 95, XP002938171
- DHAHBI J.M.: 'Dietary energy tissue-specifically regulates endoplasmic reticulum chaperone gene expression in the liver of mice' JOURNAL OF NUTRITION vol. 127, no. 9, September 1997, pages 1758 - 1764, XP002938172

## Description

### Background of the Invention

### Field of the Invention

For years, researchers have attempted to identify biomarkers of aging to facilitate the identification of interventions that might slow or reverse the aging process. Dietary calorie restriction (CR) is the only well-documented method for extending life span in homeothermic vertebrates, and is the most effective means known for reducing cancer incidence. Although many of the physiological consequences of CR were described 65 years ago, there is no consensus regarding its mode of action. Consequently, there has been no practical method of identifying interventions that might mimic such calorie-restriction effects. Rather, a researcher would have to wait the test animal's lifetime to determine whether a particular intervention impacted life-span and/or cancer incidence.

### Description of the Related Art

Mammals seem to share a common set of genes, and yet they have widely differing life spans. It is impossible to know at present whether the differences in life spans are due to differences in the sequence of specific genes, or to differences in their expression. However, it is clear from many years of study in dozens of laboratories that long term reduction in, dietary calorie consumption (CR) delays most age-related physiological changes, and extends life span in all species tested, provided malnutrition is avoided (Weindruch, et al. The Retardation of Aging and Disease by Dietary Restriction (Charles C. Thomas, Springfield, II, 1988)). These studies also have shown that CR is the most effective means now known for reducing cancer incidence and increasing the mean age of onset of age-related diseases and tumors in homeothermic vertebrates (Weindruch et al. (1982) Science 215: 1415). Thus, it seems clear that life spans can be extended through a relatively simple dietary regimen. However, there are no studies on the effects of short term calorie restriction on metabolism and gene expression.

One report has been published of gene expression profiling in muscle (Lee et al. (1999) Science 285: 1390) In these studies, many age related changes in muscle gene expression appeared to be prevented or reversed by CR. The expression profiles of 6500 genes were compared among old, long-term CR and control mice, and young control mice. Some age-related changes in muscle gene expression appeared to be wholly or partially prevented by CR.

### Summary of the Invention

The present invention contemplates a method of identifying interventions within a short time frame that mimic the effects of calorie restriction. Such interventions will lead to increased life span, reduce cancer incidence, and/or increase the age of onset of age-related diseases and tumors.

In a preferred embodiment a method of identifying an intervention that mimics the effects of caloric restriction in cells is disclosed, comprising the steps of :
exposing a biological sample comprising cells in vitro or a non-human mammal to an intervention;
waiting a specified period of time:
assessing changes in gena expression levels, levels of RNA, protein, or protein activity levels related to one or more biomarkers of aging: and
identifying said intervention as one that mimics the effects of caloric restriction if one or more changes in said levels also occurs in short term caloric restriction.

The biological sample may be either in vitro or in vivo. In a preferred embodiment, the biological sample comprises cells. In a more preferred embodiment, the cells are obtained from a mammal. In an even more preferred embodiment the mammal is a mouse.

In one embodiment, the change in gene expression levels, levels of RNA, protein, or protein activity levels corresponds to a change in gene expression for a gene encoding a chaperone protein. In a preferred embodiment, the chaperone protein is GRP78.

In one embodiment said biomarker of aging is apoptosis. In another preferred embodiment, said biomarker is aging. In another preferred embodiment, the biomarker of aging is a production of cancer cells. In another preferred embodiment, the biomarker of aging is production of stress proteins and chaperones. In another preferred embodiment, the biomarker of aging includes genes for the inflammation response. In another preferred embodiment, the biomarker of aging includes transmembrane channels/transporter proteins. In another preferred embodiment, the biomarker of aging includes genes for transcription factors. In another preferred embodiment, the biomarker of aging is amylase 2.

In a preferred embodiment, the changes in said gene expression level, levels of RNA, protein, or protein activity levels related to one or more biomarkers of aging occur in 6 weeks or less. In a more preferred embodiment, the changes in said gene expression levels, levels of RNA, protein, or protein activity levels related to one or more biomarkers of aging occur in four weeks or less. In an even more preferred embodiment, the chances in said gene expression levels, levels of RNA. protein, or protein activity levels rotated to one or more biomarkers of aging occur in two weeks or less. In a most preferred embodiment, the changes in said gene expression levels, levels of RNA, protein, or protein activity levels related to one or more biomarkers of aging occur in about two days or less.

In a one embodiment, changes in gene expression are evaluated using a gene chip. In a preferred embodiment, the gene chip contains genes for stress proteins and chaperones. In another preferred embodiment, the gene chip contains genes for the inflammation response. In another preferred embodiment, the gene chip contains genes associated with apoptosis. In another preferred embodiment, the gene chip contains genes for transmembrane channels/transporter proteins. In another preferred embodiment, the gene chip contains genes for transcription factors. In another preferred embodiment, the gene chip contains genes for amylase 2.

In an alternate embodiment, a non-human animal is used in place of the biological sample. In a preferred embodiment, the test animal is a test animal having identifying characteristics of a long-term calorie-restricted animal wherein the reference animal is an older test animal that has been on a calorie restricted diet for less than about 6 weeks and wherein said changes are used in said identifying said intervention as one that mimics the effects of calorie restriction. In a more preferred embodiment, the reference animal has been on a calorie restricted diet for less than about 4 weeks. In an even more preferred embodiment, the reference animal has been on a calorie restricted diet for less than about 2 weeks.

In a preferred embodiment, the test animal is a mouse. In a preferred embodiment, changes in gene expression are assessed in the test animal.

In a more preferred embodiment, the disclosed method further comprises:
obtaining a gene expression profile from a calorie-restricted reference animal;
comparing changes in gene expression for the test animal to the gene expression profile of the calorie-restricted reference animal; and
identifying said intervention as one that mimics the effects of calorie restriction if the gene expression profile of the test animal is statistically similar to the gene expression profile of the short term calorie restricted animal.

In a more preferred embodiment, the gene expression profile of the test animal is determined to be statistically similar to the gene expression of the calorie restricted animal by one-way ANOVA followed by Fisher's test (*P* < 0.05).

A system is disclosed for identifying an intervention that mimics the effects of calorie restriction in a test animal comprising a test animal and a gene chip comprising genes known to have altered expression during calorie restriction. The gene chip may comprise genes selected from the group consisting of genes for stress proteins/chaperones, apoptosis, inflammation response, transmembrane channels/transporter proteins, transcription factors and amylase 2.

For purposes of summarizing the invention and the advantages achieved over the prior art, certain objects and advantages of the invention have been described above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

Further aspects, features and advantages of this invention will become apparent from the detailed description of the preferred embodiments which follow.

### Brief Description of the Drawings

These and other feature of this invention will now be described with reference to the drawings of preferred embodiments which are intended to illustrate and not to limit the invention.

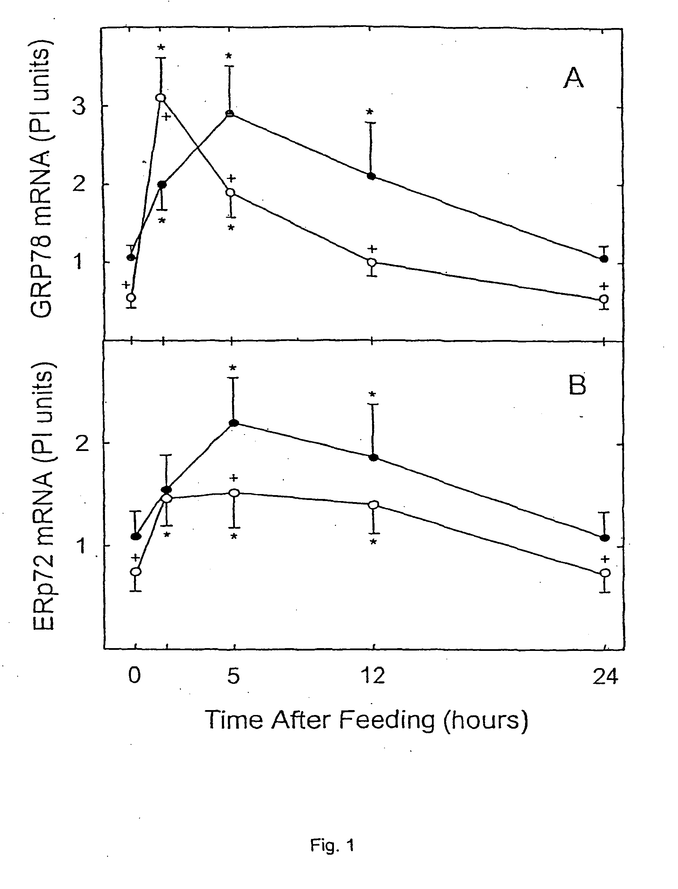

FIG. 1. Effects of feeding on hepatic GRP78 and ERp72 mRNA. At 0, 1.5, 5 and 12 h following feeding, 5 mice from each dietary group were killed. Their weights after 24 h of fasting were 22.96 ± 1.49 for CR and 37.12 ± 1.19 g for control mice. GRP78 mRNA (A) and ERp72 mRNA (B) from control (closed circle) and CR (open circle) mice were quantified using dot-blots. RNA loading and transfer were normalized using data obtained from serial probings for 18S ribosomal RNA and S-II mRNA. Similar results were obtained with both control probes. CR and control mice, fed once daily for 30 days, were fasted for 24 hours and killed (n-5, 0 time point) or refed and killed at the times specified (n=5 for each time, point). + represents P < 0.01 significance of difference between CR and control at each time paint. * represents *P* < 0.01 significance of difference from the 0 time point within each dietary group. The 0 and 24 hour times points are the same data set.

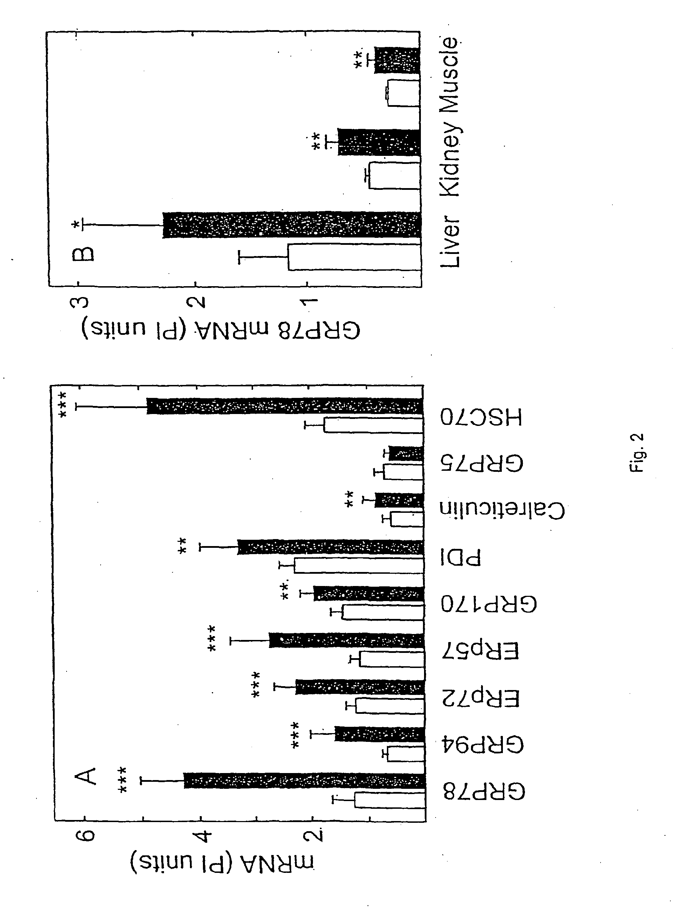

FIG. 2. The gene and tissue specificity of the chaperone feeding response. A, The domain of chaperone genes responsive to feeding was determined by quantifying hepatic chaperone mRNA abundance using RNA from mice fasted for 48 hours (n-6; open bars) or from mice fasted 48 hours, refed and killed 1.5 h later (n-6; filled bars). The mRNAs were quantified by dot-blotting and Northern blotting. There was no significant difference in the results obtained with either technique. The dot-blotting results are shown. B, Liver, kidney, and muscle GRP78 mRNA from 24-hour fasted mice (n-4), and from 24-hour fasted mice 1.5 hours after feeding (n-5). These data were from different mice than used in panel A. The statistical significance of the results are indicated (*, *P*<0.05; **, *P*<0.01; ***, *P*< 0.001).

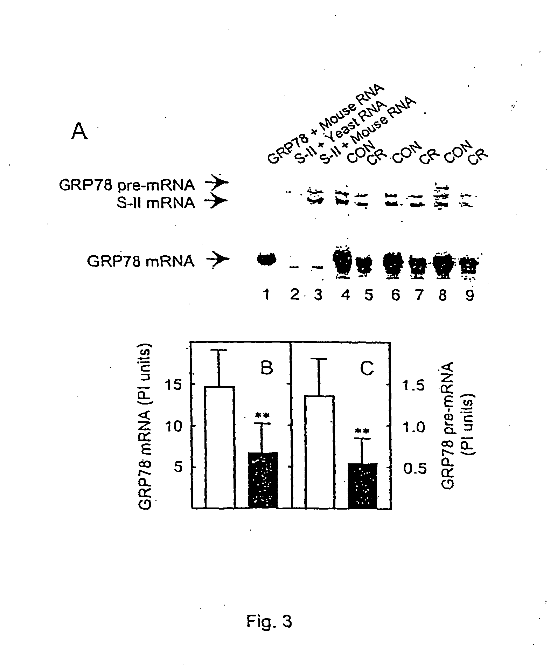

FIG. 3. Effects of CR on hepatic pre-mRNA and GRP78 mRNA abundance. A, RNase protection of pre-mRNA and mRNA in CR and control mice. Hepatic RNA was purified from control and CR mice and hybridized with an RNA probe for transcripts spanning the third intron and fourth exon boundary of the GRP78 gene. The precursor mRNA protected a 223 base region of the probe, labeled GRP78 pre-mRNA, while the GRP78 mRNA protected a 113 base fragment, so labeled in the figure. A probe for S-II mRNA coding sequences was included in each reaction as an internal control. It protected a 185 base fragment labeled S-II mRNA in the figure. Lane 1 shows the protected fragments produced by the GRP78 probe and mouse liver RNA. Lane 2 shows the fragments produced by the S-II probe hybridized to yeast total RNA. Lane 3 shows the results produced by the S-II probe hybridized to mouse liver RNA. Lanes 4, 6, and 8 show the results produced by hepatic RNA from control mice. Lanes 5, 7, and 9 show the results with RNA from CR mice. Quantification of the abundance of the protected fragments representing the GRP78 mRNA (B) and pre-mRNA (C). Studies such as those shown above were conducted using hepatic RNA from 6 CR and 6 control mice. The intensity of the protected fragments was quantified with a phosphorimager. The intensities of the pre-mRNA and mRNA fragments were normalized to the intensity of the protected fragment representing S-II mRNA. Statistical significance is indicated as in the legend to Fig. 2.

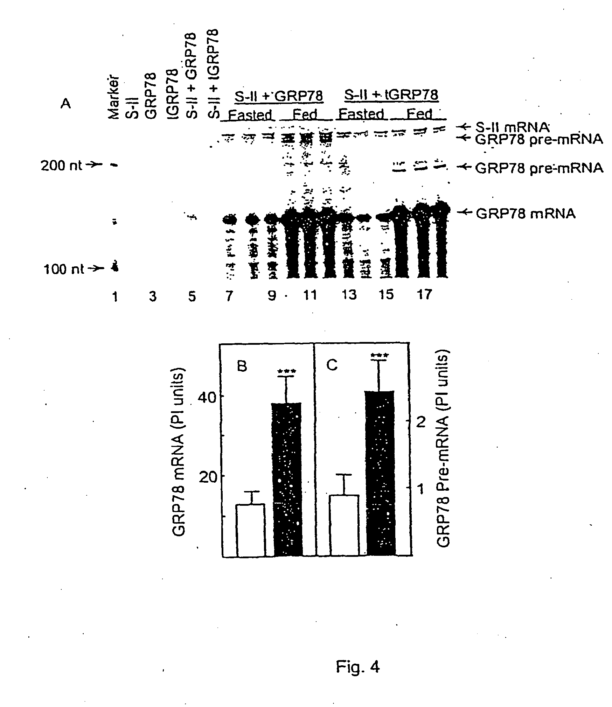

Fig. 4. Effects of feeding on hepatic GRP78 mRNA and pre-mRNA abundance. A, RNase protection of probes for hepatic GRP78 pre-mRNA and mRNA in mice after 48 hours of fasting (n=5), or 1.5 h after feeding of 48-hour fasted mice (n-5). RNA purified from liver was hybridized either to a probe for primary-transcripts containing the exon 7 and intron 7 boundary of the GRP78 gene which produced a 257 base protected fragment (labeled S-II + GRP78; lanes 7.12), or to a probe for primary transcripts spanning the exon 7 and intron 7 boundary, which protected a 200 nucleotide fragment (labeled S-II + tGRP78, lanes 13-18), as indicated in the figure. GRP78 mRNA produced a 143 nucleotide fragment representing GRP78 mRNA, as indicated in the figure. A probe for S-II mRNA coding sequences was included in each reaction as an internal control. With this probe, S-II mRNA protected a 277 nucleotide fragment, labeled S-II mRNA in the figure. Lane 1, RNA markers. Lanes 2-6, hybridization of the indicated probes with yeast tRNA. Lanes 7-12, hybridization of the GRP78 and S-II probes with RNA from fasted (lanes 7-9) and refed (lanes 10-12) mice. Lanes 13-18, hybridization of tGRP78 and S-II probes with RNA from fasted (lanes 13-15) and refed (lanes 16-18) mice. Quantification of the abundance of the protected fragments representing the GRP78 mRNA (B) and pre-mRNA (C). Studies such as those shown above were conducted using hepatic RNA from 6 CR and 6 control mice. The intensity of the protected fragments was quantified and normalized as described in Fig. 3 above. Statistical significance is indicated as in the legend to Fig. 2.

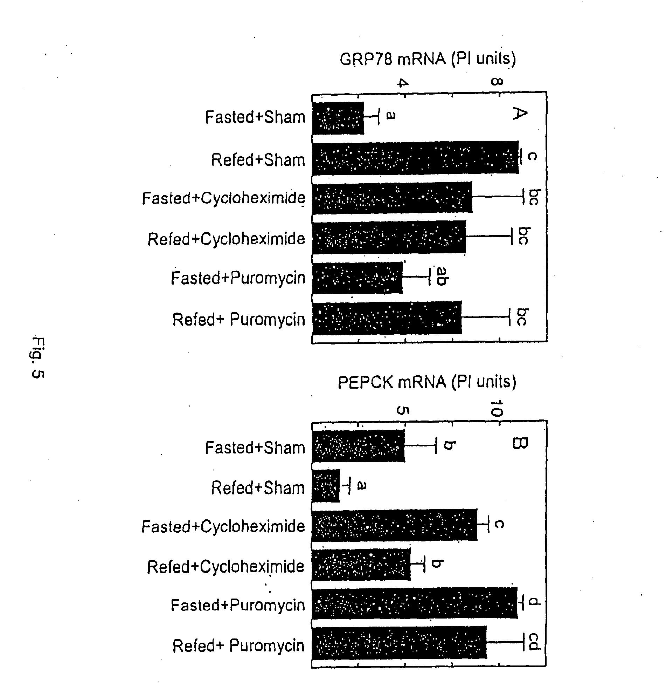

FIG. 5. Effects of protein synthesis inhibitors on the feeding response of GRP78 (A) and PEPCK (B) mRNA. Mice fasted for 48 h were injected i.p. with vehicle and after 1 hour injected a second time i.p with vehicle (Refed+Sham; n-6). Mice fasted for 48 hours were injected i.p. with vehicle 30 min before and 30 min after feeding (Refed+Sham, n=6). Mice fasted for 48 h were injected i.p. with cycloheximide and after 1 hour injected a second time i.p with cycloheximide (Fasted+Cycloheximide; n=6). Mice fasted for 48 h were injected i.p. with cycloheximide 30 min before and 30 min after feeding (Refed+Cycloheximide; n=6). Mice fasted for 48 h were injected i.p. with puromycin and after 1 hour injected a second time i.p with puromycin (Fasted+Puromycin; n-6). Mice fasted for 48 h were injected i.p. with puromycin 30 min before and 30 min after feeding (Refed+Puromycin; n-6). GRP78 and PEPCK mRNA abundance were determined using purified hepatic RNA. Bars without common superscripts are significantly different (*P*< 0.005).

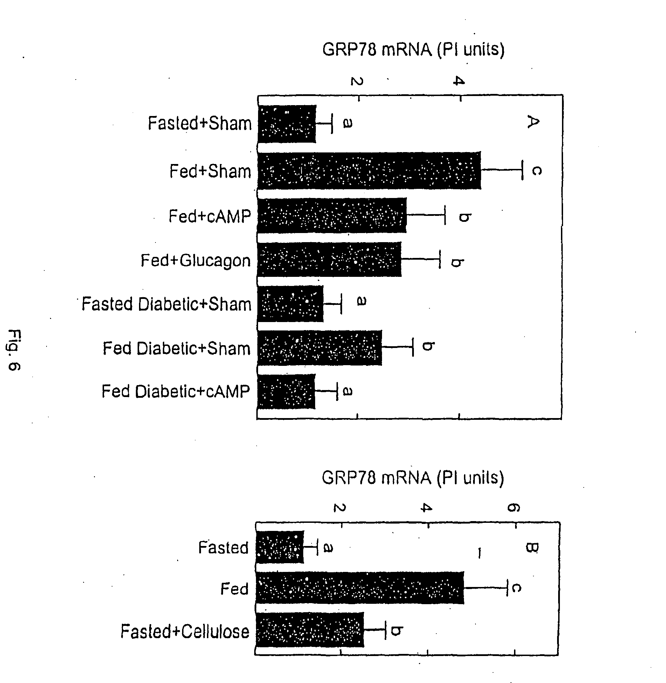

FIG. 6. Regulation of the fasting-feeding response by insulin, dibutyryl-cAMP, glucagon, and ingestion of mineral oil and cellulose. A, Groups of six mice were fasted for 48 h and treated as follows: Fasted +Sham mice were injected with vehicle and 1 h later vehicle injected a second time; Fed + Sham mice were sham injected with vehicle 30 min before and 30 min after feeding; Fed+cAMP mice were injected with dibutyryl-cAMP and theophylline 30 min before and 30 min after feeding; Fed+glucagon mice were injected with glucagon 30 min before and 30 min after feeding; Fasted Diabetic+Sham mice, previously rendered diabetic with STZ, were vehicle injected and 1 h later vehicle injected a second time; Fed Diabetic+Sham, STZ-diabetic mice were sham injected with vehicle 30 min before and 30 min after feeding; Fed Diabetic+cAMP, diabetic mice were injected with dibutyryl-cAMP and theophylline 30 min before and 30 min after feeding. All mice were killed 1 h after their last injection. Total RNA was isolated from the liver and subjected to dot-blot analysis. Bars with no common superscripts are significantly different (*P*< 0.005). B, Effects of mineral oil and cellulose ingestion on liver GRP78 mRNA abundance. Groups of six mice were fasted for 48 h and treated as follows: Fasted, mice were fasted for 48 h and killed; Fed, mice were fasted for 48 h, fed, and killed 1.5 h later; Fasted+cellulose, mice fasted for 48 h were fed a mixture of cellulose and mineral oil, and killed 1.5 h later. Significance is indicated as in the legend to Fig. 5.

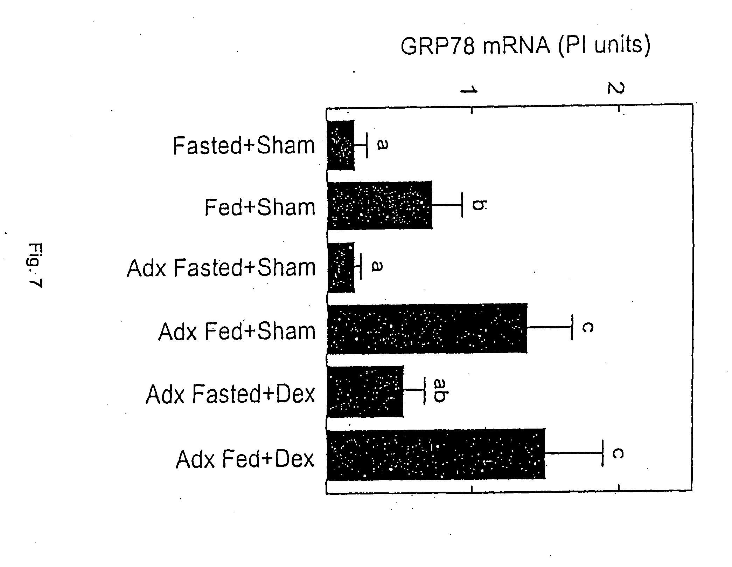

FIG. 7. Effects of adrenalectomy and dexamethasone administration on the expression and regulation of hepatic GRP78 mRNA. Groups of six mice were fasted for 48 h and treated as follows: Fasted+Sham, sham-operated mice were injected with vehicle IP 7.5 h and 1.5 h before they were killed; Fed+Sham, sham-operated mice were injected with vehicle IP 6 hours before and 30 min after feeding, and mice were killed 1 h after the last injection; Adx Fasted+Sham, adrenalectomized mice were injected with vehicle IP 7.5 h and 1.5 h before they were killed; Adx Fed +Sham, adrenalectomized mice were injected with vehicle IP 6 hours before and 30 min after feeding, and the mice killed 1 h later; Adx Fasted+Dex, adrenalectomized-mice were injected IP with dexamethasone 7.5 h and 1.5 h before they were killed; Adx Fed+Dex, adrenalectomized mice were injected IP with dexamethasone 6 hours before and 30 min after feeding, and killed 1 h later. Significance is indicated as in the legend to Fig. 5.

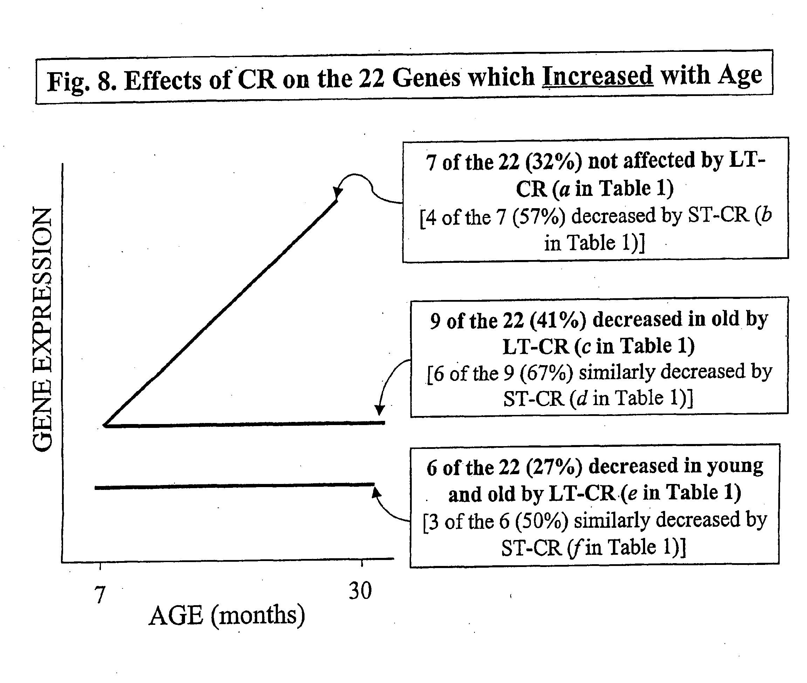

FIG. 8. Effects on CR on the 22 genes which increased with age. The upper line represents the 7 genes which were not affected by LT-CR. The middle line represents the 9 genes which decreased in old by LT-CR. The bottom line represents the 6 genes which decreased in young and old by LT-CR.

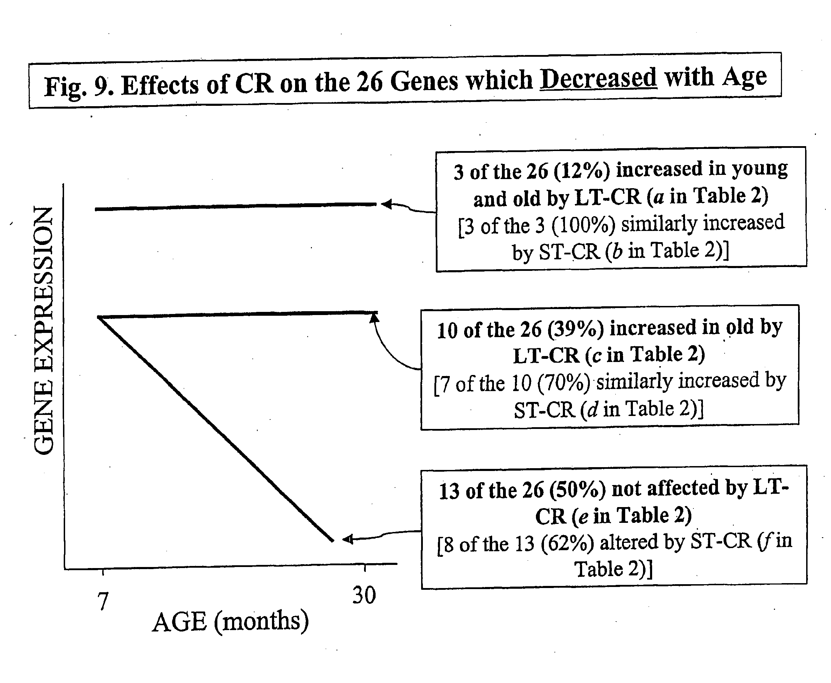

FIG. 9. Effects on CR on the 26 genes which decreased with age. The upper line represents the 3 genes which increased in young and old by LT-CR. The middle line represents the 10 genes which increased in old by LT-CR. The bottom line represents the 13 genes which were not affected by LT-CR.

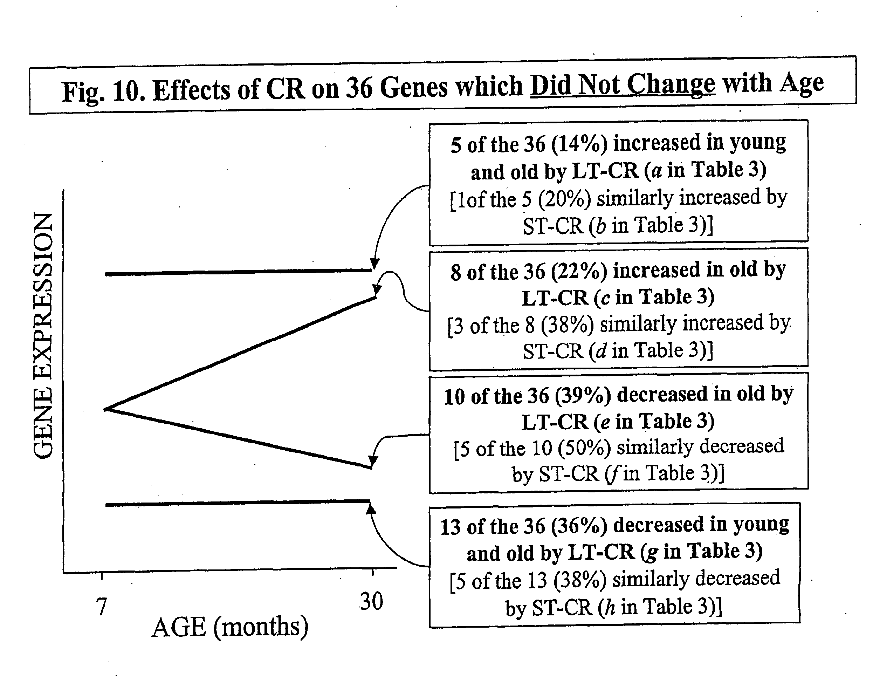

FIG. 10. Effects of CR on 36 genes which did not change with age. The top line represents the 5 genes which increased in young and old by LT-CR. The second from top line represents the 8 genes which increased in old by LT-CR. The third from top line represents the 10 genes that decreased in old by LT-CR. The bottom line represents the 13 genes that decreased in young and old by LT-CR.

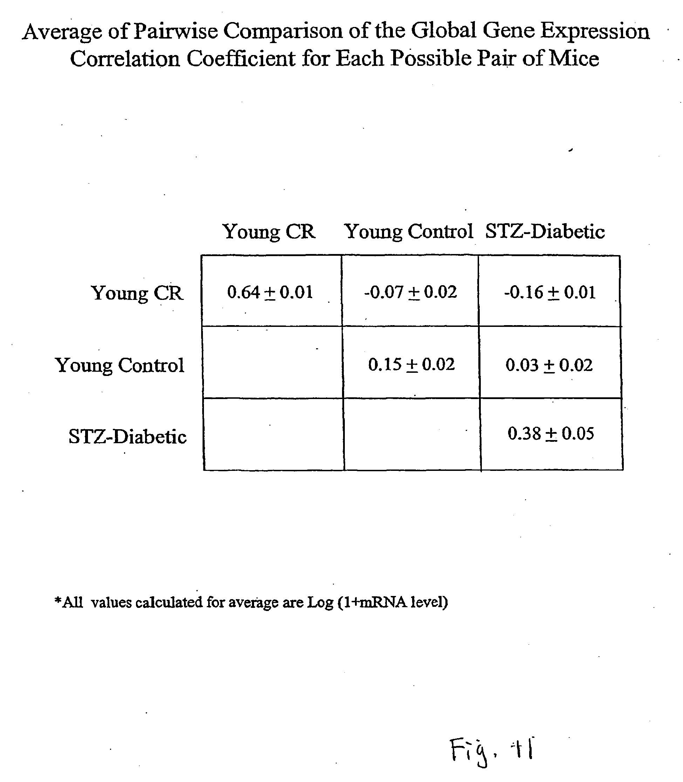

Fig. 11. Average of pairwise comparison of the global gene expression correlation coefficient for each possible pair of mice.

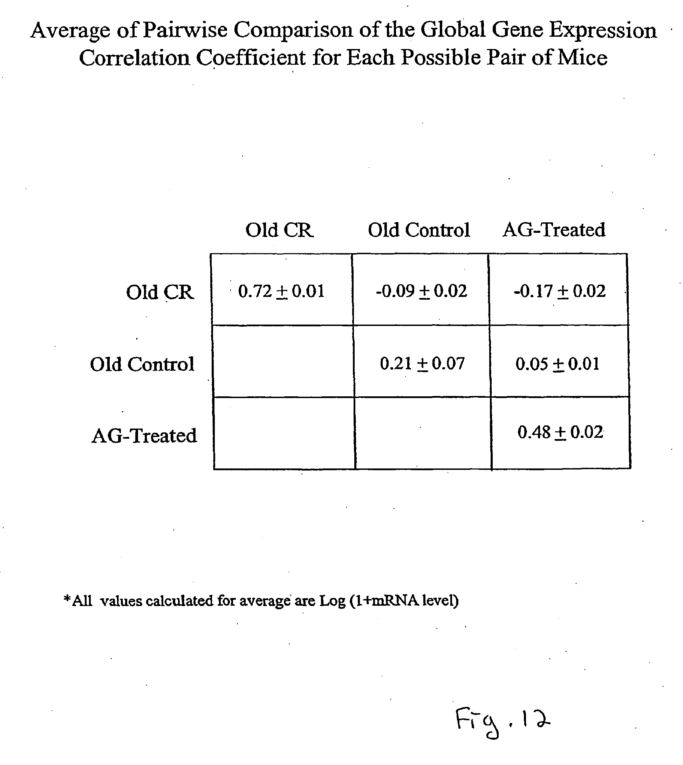

FIG. 12. Average of pairwise comparison of the global gene expression correlation coefficient for each possible pair of mice.

### Detailed Description of the Preferred Embodiment

While the described embodiment represents the preferred embodiment of the present invention, it is to be understood that modifications will occur to those skilled in the art without departing from the spirit of the invention. The scope of the invention is therefore to be determined solely by the appended claims.

The effects of long term calorie restriction include increases in the rate of clearance of serum proteins, including glucose damaged serum proteins, from the blood as well as changes in gene expression. For example, long term calorie restriction affects expression of stress proteins/ chaperones, genes involved in apoptosis, the inflammation response, transmembrane channels and transporter proteins, transcription factors and others.

Molecular chaperones assist in the biosynthesis, folding, processing, and degradation of proteins. Many of the chaperone genes are stress inducible. Subsets of chaperones are induced by different physiological stressors. For example, the majority of the known endoplasmic chaperones are induced by stresses that produce malfolded or improperly glycosylated proteins in the ER. This unfolded protein response pathway also may adjust the level of protein trafficking through the ER to the level of ER chaperones. Other chaperones, such as the abundant cytoplasmic chaperone HSC70 are normally thought of as constitutively expressed. The present invention is based in part on the finding that certain chaperone genes are down regulated by calorie restriction (such regulation is thought to be mediated through the insulin and glucagon pathways). The expression of Erp72, Erp57, GRP 170, GRP78, GRP94, HSC70, Calnexin, and Calreticulin are particularly affected by calorie restriction.

The fasting mRNA and protein levels of nearly every ER chaperone studied were found to be significantly and consistently reduced in the livers of CR mice chronically fed a low calorie diet. In the case of GRP78, levels decreased by approximately 66%. Further, the reduction in chaperone mRNA levels was proportional to the reduction in calorie consumption. The fewer calories consumed, the lower the level of chaperone mRNA. We subsequently found that fasting chaperone mRNA levels changed over the course of 2 weeks in response to different levels of chronic calorie consumption. The more calories consumed per week, the higher the chaperone levels. Chaperone mRNA levels respond more rapidly to calorie consumption.

mRNA for most ER chaperones, and for the major cytoplasmic chaperone, HSC70, are dynamically responsive (within 1.5 h) to each meal, and to the number of calories consumed. Features of this induction distinguish it from the unfolded protein response. The feeding induction was observed in kidney and muscle tissue, as well as in the liver. Postprandial changes in glucagon, in conjunction with insulin, were found to be the key mediators of this induction.

Chaperone mRNA abundance responds within 1.5 h to caloric intake. Insulin and glucagon may be important for the response. This feeding response is rapid. By 1.5 hours after feeding, ER chaperone mRNAs were at or near their maximum level of induction. This feeding-related induction is not limited to one strain of mouse or to one species. Further, the response is found in tissues other than liver. Thus, it is a response which is generally important to the physiology of a variety of cell types in vivo.

Because many chaperones are relatively stable proteins, their protein levels change more slowly in response to caloric intake than their mRNAs. For example, GRP78 protein has a half-life of over 24 hours in cultured cells. We found that GRP78 protein levels change only over a span of several days in response to changes in average daily calorie consumption. In this way, many chaperones may effectively integrate the rapid mRNA responses to feeding into longer term changes in chaperone protein levels. Long term differences in average calorie consumption do lead to differences in the hepatic levels of both ER and some cytoplasmic chaperones.

RNase protection assays indicate that GRP78 mRNA is transcriptionally regulated in response to feeding. Similar RNase protection results were obtained with hepatic RNA from chronically CR mice. Thus, both feeding and CR transcriptionally alter the expression of the chaperone genes.

Puromycin led to partial induction of GRP78 mRNA. It is unlikely that induction of the mRNA by cycloheximide is due to stabilization of the transcript by polysome aggregation. While cycloheximide protects some mRNAs from inactivation and degradation in this way, puromycin does not. Rather, it inhibits translation by polysome dissociation. Thus, maintenance of low hepatic GRP78 mRNA levels most likely requires the action of an unstable repressor of GRP78 gene expression in fasted mice. In the presence of inhibitors of translation, this repressor may decay, releasing the gene from repression.

Second, there was no augmentation of GRP78 mRNA induction when feeding and inhibition of translation were combined. While partial induction of the mRNA was found in puromycin treated mice, feeding induced the mRNA to the same level found in the absence of the inhibitor. Further, cycloheximide induced the mRNA to the same extent. Without being bound to any particular mechanism, it is suggested that the inhibitors and feeding may induce the gene through a common pathway.

Third, since feeding fully induced GRP78 mRNA in puromycin treated mice, de novo protein synthesis is not required for the feeding response. Preexisting signaling and regulatory factors mediate the response. Fourth, the feeding response cannot result from a postprandial increase in protein trafficking through the ER. Enhanced ER de novo protein trafficking can induce chaperone mRNA. However, no such increase could have occurred in the presence of puromycin.

Fifth, the unfolded protein and growth factor responses are not involved in the induction of chaperones by feeding. Cycloheximide blocks the unfolded protein and growth factor responses. We are aware of only one manipulation besides feeding capable of inducing ER chaperone mRNA in the presence of cycloheximide. GRP mRNAs are induced by cellular hypoxia in culture, and this induction is independent of cycloheximide treatment. Whether the feeding and hypoxia response share common molecular pathways is unknown at present.

Feeding is well-known to decrease glucagon and increase insulin levels. Both glucagon and dibutyryl-cAMP blunted the feeding induction of GRP78 mRNA. Thus, glucagon is a negative regulator of GRP78 expression in vivo. The feeding induction of GRP78 mRNA was significantly reduced in STZ-diabetic mice. Without being bound to any particular mechanism, this result, and the absence of a feeding response in STZ-diabetic, dibutyryl-cAMP-treated mice indicate that the action of both hormones is required for the response.

Other effectors which are known to respond to feeding were also examined. Luminal stimuli can promote the release of gastrointestinal hormones. For this reason, we determined whether luminal filling with a non-digestible mixture of mineral oil and cellulose could stimulate chaperone expression. A small but significant response was found. However, insulin and glucagon have a much stronger effect on chaperone mRNAs, indicating they are the signals primarily responsible for the feeding response.

The feeding response was enhanced in adrenalectomized mice. These results suggest that other adrenal hormones, perhaps catecholamines, may partially blunt the chaperone mRNA response to feeding. However, the mechanism by which these hormones stimulate the feeding response is unknown at present.

Overall, feeding rapidly and strongly induced the mRNA for the major cytoplasmic chaperone, HSC70, and most ER chaperones examined. Feeding also induced ER chaperone mRNAs in at least three different tissues. Feeding and CR regulated chaperone mRNA abundance at the transcriptional level. Without being bound to any particular mechanism, feeding appeared to release chaperone gene expression from the effects of an unstable inhibitor. Insulin was required, and glucagon and cAMP mediated the feeding response. Postprandial changes in glucagon levels may be the primary mediator of the response. Gastrointestinal and adrenal hormones, but not glucocorticoids also have a role in the feeding response.

Surprisingly, changes in gene expression are also observed with short-term calorie restriction. These changes in gene expression are similar to the changes observed-in long-term CR. Short-term calorie restriction occurs when switching a mature test animal to a diet which is about 50% less than a control diet for about 2-6 weeks. In a preferred embodiment, the test animal is a mature mouse and the mature mouse is switched to a calorie-restricted diet at about 31 months. Preferably, an intermediate diet which is about 20. 40% less than a control diet is employed for about two weeks before switching to a CR diet for an additional two weeks.

Both long term and short-term CR produces its profound effects on mammalian physiology by affecting the expression of genes. To identify as broadly as possible the effects of caloric restriction on global patterns of gene expression, gene chip technology was utilized to characterize the effects of long and short term CR on the expression of approximately 11,000 mouse genes in the liver.

Liver is an attractive organ for study, since it contains a number of cell types, allowing assessment of the effects of CR on hepatocytes, which are primarily responsible for the regulation of metabolism and blood sugar, neurons of the enteric nervous system, immune system cells in the blood, and vascular smooth muscle cells, among others.

The methods of the present invention include the identification of interventions that mimic the effects of short term calorie restriction. Particularly contemplated by the invention are methods of identifying interventions that have an effect on life span, aging, and/or the development of age-related diseases and cancer.

In certain embodiments, such methods comprise exposing cells to an intervention, and observing whether the intervention affects the gene expression profile, levels of RNA, protein, or protein activity related to one or more biomarkers of aging. Preferably, such changes in gene expression, RNA, protein, or protein activity levels would occur within four weeks of the intervention. More preferably, such changes would occur within two weeks of the intervention, and most preferably, such changes occur within two days of the intervention. Such methods permit the identification of pharmacological or other means of achieving a metabolic state similar to the profile observed with short-term CR.

The methods of the present invention include the use of in vitro assays (including gene chip assays) as well as animal assays. Preferably, however, the methods are carried out in live mammals. For example, transgenic mice having enhanced chaperone expression may be used to measure an intervention's ability to reduce cancer, apoptosis, and/or life span. Alternatively, the present methods may be used to identify interventions that mimic short term calorie restriction simply by measuring the intervention's ability to alter gene expression for a particular gene or set of genes in live mammals. Such methods allow identification of effective interventions in a short period of time. Interventions identified by the methods of the present invention may be pharmacological, surgical or otherwise. Combinatorial chemistry may also be used in order to screen a large number of pharmacological compounds. In general, the interventions identified by the present invention should be effective in the treatment of cancer, diabetes, age-related diseases and/or the extension of life span.

While the described embodiment represents the preferred embodiment of the present invention, it is to be understood that modifications will occur to those skilled in the art without departing from the the invention. The scope of the invention is therefore to be determined solely by the appended claims.

### EXAMPLES

### Example 1

### Long Term Calorie Restricted (LTCR) Animals and Treatments for Chaperone Studies

Female, 28-month old mice of the long-lived F, hybrid strain C3B10RF, have been described previously. Mice were weaned at 28 d, housed individually and subjected to one of two diets. The control diet consisted of casein (high protein), 207.0 g/kg, OL-methionine. 4.0 g/kg. dextrose monohydrate, 301.8 g/kg, corn starch, 290.0 g/kg. cellulose, 702. g/kg, brewer's yeast, 8.0 g/kg, Harlan Teklad Vitamin Mix #40060, 10.0 g/kg, Harlan Teklad AIN-76 Mineral Mix #170915, 35.0 g/kg, calcium carbonate (CaCO₃), 3.0 g/kg, magnesium oxide (MgO), 1.0 g/kg, sodium fluoride (NaF), 2.3 mg/kg, sodium molybdate (Na2MoO·2H₂O), 0.5 mg/kg. The 50% restricted diet consisted of casein (high protein), 362.0 g/kg, OL-methionine, 7.0 g/kg. dextrose monohydrate, 172.03 g/kg, corn starch, 153.1 g/kg, cellulose, 83.6 g/kg, brewer's yeast, 14.0 g/kg, Harlan Teklad Vitamin Mix #40060, 17.5 g/kg, harlan Teklad AIN-76 Mineral Mix #170915, 61.25 g/kg. calcium carbonate (CaCO₃), 5.25 g/kg, magnesium oxide (MgO), 1.75 g/kg, sodium fluoride (NaF), 3.0 mg/kg, sodium molybdate (Na2MoO·2H₂O), 0.9 mg/kg. From weaning, control mice were fed 4.8 g of the control diet on Monday through Thursday. On Friday they were fed 13.8 g of control diet. This feeding regimen provided 450 kJ/wk. From weaning, the 50% calorie restricted (CR) mice were fed 4.6 g of the restricted diet on Monday and Wednesday, and 6.9 g on Friday. This regimen provided 225 kJ/wk. Each dietary group received approximately equal amounts of protein, corn oil, minerals and vitamins per gram body weight. The amount of carbohydrates consumed varied between groups. Beginning 30 d before these studies, the control mice were fed 4.1 g (54.44 kJ) control diet daily at 0900 h. The 50% restricted mice were fed 2.3 g of restricted diet (32 kJ) daily at 0900 h. During this 30 d period, the control and restricted mice received approximately 15% and 50% less dietary energy than normally thought to be required for a typical mouse {Subcommittee on Laboratory Animal Nutrition & Committee on Animal Nutrition 1978 ID: 5480} All food was routinely consumed within 30 min.

Retired male Swiss-Webster breeder mice were purchased from Jackson Laboratories. Beginning 30 days before the studies, the mice were fed Monday and Wednesday 11 g and Friday 16.6 g of the control diet daily at 0900 h. In fasting-feeding studies, mice were deprived of food for 48 h, fed 5.5 g of the control diet at 0900 h, and killed 90 min later. The food was consumed within 30 min. Diabetes was induced by three weekly intraperitoneal injections of streptozotocin [10 mg/100 g body weight (b.w.)] in 50 mM sodium citrate, pH 4.5. Mice were diabetic one week after the last injection. Only mice with blood glucose level higher than 3 mg/ml were used. Mice injected with equivalent volumes of sodium citrate served as controls for the STZ-diabetic mice. Adrenalectomized and sham-operated mice were purchased from Jackson Laboratories. Dibutyryl cAMP (Sigma; 18 mg.100 g b.w.), and theophylline (Sigma; 3 mg/100 g b.w), glucagon (Sigma; 300 µg/100 g b.w.), dexamethasone (Sigma; 125 µg/100 g b.w), cycloheximide (Sigma; 4 mg.100 g b.w.), and puromycin (Sigma; 10 mg.100 g b.w.), were administered intraperitonealy to mice as specified in the figure legends. Mice received two doses of each drug or drug combination. The first injection was administered 30 min before feeding, and the second injection was administered 30 min after feeding. Mice were killed 1.5 h after the start of feeding. Drug-injected mice consumed similar amounts of food as control animals during the feeding period. All animal use protocols were approved by the institutional animal use committee of the University of California, Riverside.

### Example 2

### RNA Isolation and Quantification for Chaperone Studies

Mice were killed and the livers, kidneys, and muscle were removed. Muscle from the hind legs and back was removed and pooled for each animal. Tissues were flash frozen in liquid nitrogen. Approximately 0.2 g of frozen tissue was homogenized for 40 s in 4 ml of TRI Reagent (Molecular Research Center, Cincinnati, OH) using a Tekmar Tissuemizer (Tekmar, Cincinnait, OH) at a setting of 55. RNA was isolated as described by the TRI Reagent supplier. RNA was resuspended in FORMAzol (Molecular Research Center) and Northern and dot blots were performed using 20 and 10 µg of RNA respectively. The RNA was analyzed using Northern blots to verify its integrity. Dot blots were used to quantify mRNA levels (24; 27). Specific mRNA levels were normalized to the level of total RNA and/or mRNA present in each sample using hybridization with radiolabeled complementary DNA to 18S rRNA and/or transcription factor S-II, as indicated in the figure legends (12; 27). The murine ERp72 2.5 kb cDNA was excised with *Bam*HI from pcD72-1 (19). The 1235 bp murine GRP75 coding fragment was excised with *Hind*III from pG7z-PBP1.8 (6). A 1.5 kb coding fragment of GRP78 cDNA was produced by digestion of p3C5 with *Eco*RI and *Pst*I (15). A 1.4 kb hamster GRP94 coding fragment was produced by *Eco*RI and Sa/K digestion of p4A3 (15). A 664 bp coding fragment of rat calreticulin (nucleotides 148 to 812) was produced by PCR from GT10.01 (23). The entire 2.4 kb cDNA of murine PDI was excised from pGEM59.4 with *Sac*I and *Bam*HI (19). A 1 kb coding fragment of hamster GRP170 cDNA was excised with *Eco*RI and *Xho*I from pCRtmII (16). The 1.9 kb cDNA of murine ERp57 was excised with *Hind*III and *Sst*I from pERp61 (18). The 1 kb cDNA of murine HSC70 was excised with *Pst*I from phsc1.5 (9). The 1.3 kb PEPCK coding fragment was produced by SphI followed by *Sal*I digestions of pGEM5ZEP (a gift from Dr. Ganner D.K. Vanderbilt University School of Medicine, Nashville, TN). The fragments were isolated by.agarose gel electrophoresis and radioactively labeled using a "QuickPrime Kit (Pharmacia) according to the manufacturer's instructions.

### Example 3

### RNase Protection Assays for Chaperone Studies

A 223 base pair (bp) DNA fragment made up of 110 bases of intron 3 and all 113 bases of exon 4 of the mouse GRP78 gene was synthesized by PCR using genomic DNA as template and inserted into pT7/T3 (Ambion, Austin, Texas). Two probes of the junction region of intron 7 and exon 7 of the GRP78 gene were produced by PCR using mouse genomic DNA as template. A 257-base fragment including all of exon 7 and the first 113 bases of intron 7 was produced. A 200-base fragment including all of exon 7 and the first 56 bases of intron 7 also was produced. The T7 RNA polymerase promoter was ligated to these PCR fragments using a Lig'nScribe kit as described by the supplier (Ambion). These constructs were used as template for the synthesis of [³²P]-labeled antisense RNA probes using a MAXIScript kit as described by the supplier (Ambion). RNase protection assays were performed using an RPA II kit as described by the supplier (Ambion). Hybridization of the 257-base RNA probe with GRP78 pre-mRNA protected all 257-bases corresponding to exon 7 and the first 113 bases of intron 7. Hybridization of the 200-base RNA probe to pre-mRNA protected 200-bases corresponding to all of exon 7 and the first 56 bases of intron 7. Hybridization of either probe to GRP78 mRNA protects the 143-bases complementary to exon 7. A 185- and a 277-bp cDNA fragment of S-II cDNA was synthesized and subcloned into pT7/T3 (12). [³²P]-labeled RNA probes for the sense and antisense transcripts were synthesized in vitro and RNase protection assays performed. Hybridization with S-II mRNA protected the entire 185· or 277-base region of the probes. Protection of only the sense strand probes was detected. Quantitation of the hybridized fragments was determined with ImageQuaNT (Molecular Dynamics, Sunnyvale, CA).

### Example 4

### Plasma Glucose and Insulin for Chaperone Studies

Plasma glucose, insulin, and glucagon concentrations were determined using Glucose [HK] 10 (Sigma, St. Louis, M0), Rat Insulin RIA and Glucagon RIA kits (Linco Research, St. Charles, M0), as described by the suppliers.

### Example 5

### Statistical Analysis for Chaperone Studies

The data shown in Figure 1 are expressed as means ± SD for 5 mice at each time point. The effects of food deprivation and subsequent feeding on mice of each dietary group were analyzed using a one-way ANOVA followed by Fisher's test. The analysis determined whether individual time point means differed from time 0 means within each dietary group. It also determined the differences between the means of the control and CR groups at each time point. Differences of P < 0.05 were considered significant. Values are expressed as means ± SD. Significance was determined with either Student's unpaired t-test (*P* < 0.95) or a one-way ANOVA followed by Fisher's or Tukey's tests (*P*< 0.01). All statistical analyses were performed with Minitab Statistical Software (Minitab, State College, PA).

### Example 6

### Chronic and Acute Effects of Calorie Consumption on Hepatic Chaperone mRNA

Feeding of the fasted mice rapidly induced the abundance of GRP78 and ERp72 mRNA (Figures 1A and 1B). A large increase in chaperone mRNA was detected by 1.5 h after feeding, the first time point studied. The 24-h fasting levels (0 time) of GRP78 and ERp72 mRNA were lower in the CR mice. The response to feeding was kinetically different in control and CR mice. Thus, the amount of food consumed affects the kinetics of the response. The integrated level of GRP78 and ERp72 mRNA over the entire 24-hour period was also less in the CR than in control mice. Similar results were obtained when the effects of feeding on HSC70, ERp57, and calreticulin mRNA were determined (data not shown). Thus, this represents a common response of chaperone gene expression to feeding.

### Example 7

### Fasting-Feeding induced Multiple Chaperone mRNAs in Multiple Tissues

Mice were fasted for 48' hours and refed for 1.5 hours. Hepatic GRP78 mRNA was induced approximately 3-fold after this time (Figure 2A). The mRNA for the other ER chaperones investigated, ERp57, ERp72, GRP94, GRP170, PDI, and calreticulin, and for the most abundant cytoplasmic chaperone, HSC70, also were induced by feeding (Figure 2A). HSC70 was induced by nearly 3-fold. No changes in the mitochondrial chaperone GRP75 was detected in this study. By examining chaperone levels in other tissues of fasted and fed mice, we found that the feeding-related chaperone induction extends to at least kidney and muscle (Figure 2B). GRP78 mRNA induction is shown in the figure (Figure 2B). HSC70 mRNA was also induced in these tissues (data not shown). In studies not shown, we have found that a similar induction of hepatic chaperone mRNAs occurs in rat. Thus, the response is shared by other species.

### Example 8

### CR Reduces the Abundance of the GRP78 Primary Transcript

RNase protection studies were used to investigate the responsiveness of the GRP78 mRNA and primary transcript to chronic differences in dietary calorie consumption. A probe was utilized for these studies designed so that the GRP78 primary transcript protected a 223 base RNA fragment representing the third intron-fourth exon boundary of the transcript (Figure 3A lane 1, upper band). The mRNA protected a 113 base fragment of the probe which represents the fourth exon of the gene (Figure 3A, lane 1, lower band). Much less of the 223 and 113 base GRP78 precursor and mRNA probes were protected by RNA from CR mice (Figure 3A, lanes 4-9). A probe for 185 bases of S-II mRNA was included in each sample as an internal control (Figure 3A, lane 3). S-II mRNA is unresponsive to CR or fasting-feeding (25). The unlabeled bands in Figure 3 represent RNase-resistant artifacts of the S-II probe (Figure 3A, lane 2).

When the amount of protected probe was quantified and normalized to the signal obtained from the S-II probe, it became clear that the abundance of the chaperone precursor and mRNA were decreased to the same extent in the CR mice (Figure 3B). The same conclusion was reached using a probe for the boundary regions of intron 7 and exon 7. Consequently, CR decreases either the rate of GRP78 gene transcription or the stability of the GRP78 primary transcript. The data are not consistent with blocked or paused GRP78 gene transcription or changes in the stability of the mRNA in CR mice.

### Example 9

### Fasting-feeding induction of the GRP78 primary transcript

RNase protection studies also were used to investigate the fasting-feeding response. RNA isolated 1.5 h after feeding protected much more of a 257 base fragment representing the exon 7-intron 7 boundary of the primary transcript than RNA isolated from fasted mice (compare Fig. 4A, lanes 10-12 to lanes 7-9). Similar results were obtained with a probe in which 200 bases representing the exon 7-intron 7 boundary were protected (compare Fig. 4A, lanes 16-18 to lanes 13-15). In each case, RNA from refed mice also protected more of the 143 base fragment representing the exon 7 region of the mRNA (Fig. 4A). A probe for 277 bp of the S-11 mRNA was present in each assay for use as an internal control.

Quantification of these data, and normalization of the S-11 internal control demonstrated that the mRNA and the precursor RNA were induced by feeding to essentially the same extent (Fig. 4B and 4C). Similar results were obtained using the probe described earlier for the third intron fourth exon boundary of the gene (data not shown). Without being bound to a specific mechanism, these data suggest the same molecular step is responsible for regulating the genetic responsiveness of chaperones to both acute and chronic changes in calorie consumption. This mechanism appears to involve changes in either the transcription or the stability of the primary transcript.

### Example 10

### Inhibitors of protein synthesis

To investigate the physiological basis for the fasting-feeding response, studies were performed using inhibitors of protein synthesis. Fasted mice were treated with a dose of cycloheximide or puromycin sufficient to inhibit greater than 95% of protein synthesis in the liver. Treatment with cycloheximide strongly induced GRP78 mRNA in fasted mice (Fig. 5A). GRP78 mRNA also was strongly induced in cycloheximide-treated, refed mice. Puromycin treatment modestly induced GRP78 mRNA in fasted mice (Fig. 5A). Feeding of puromycin treated mice fully induced the mRNA. Thus, induction by feeding does not appear to require de novo protein synthesis. Further, these results suggest that the lower chaperone mRNA levels in fasted mice may involve the action of a rapidly turning over factor.

The effects of the protein synthesis inhibitors on PEPCK mRNA also was determined as a positive control. The effects of fasting-feeding and cycloheximide treatment on this mRNA are well known. Fasting induced, and feeding repressed PEPCK mRNA, as expected (Fig. 5B). Also, as expected from published data, cycloheximide increased PEPCK mRNA in both fasted and refed mice through its effects on PEPCK mRNA stability. The effects of the inhibitors on PEPCK mRNA levels indicate the inhibitors were efficacious in these studies.

### Example 11

### Pancreatic hormones and glucose

The physiological hallmarks of the fasting-feeding transition are increased circulating insulin and decreased circulating glucagon. In the studies shown in Fig. 6, fasted and refed sham-injected mice had serum glucose concentrations of 84.4 ± 5.1 and 121.1 ± 8.0 mg/dl, serum insulin concentrations of 0.491 ± 0.203 and 1.3 ± 0.256 pmol/ml, and serum glucagon concentrations of 143 ± 22.4 and 81.4 ± 13.2 pg/ml, respectively.

To investigate whether these hormones are involved in the postprandial induction of GRP78 mRNA, the effects of cAMP, glucagon, and STZ-induced diabetes on the response were examined. Administration of either dibutyryl cAMP or glucagon reduced the response of GRP78 mRNA to feeding (Fig. 6A). Vehicle alone had no effect. Likewise, STZ-induced diabetes resulted in a blunted response to feeding although it did not modify the fasting level of GRP78 mRNA. When STZ-induced diabetes was combined with cAMP administration, the postprandial induction of GRP78 mRNA was obliterated. The mRNA remained at fasting levels Without being bound to any particular mechanism, these results suggest that glucagon, acting to increase intracellular cAMP levels, suppresses chaperone gene transcription, or possibly GRP78 pre-RNA stability. Further, they suggest that insulin is required for full responsiveness of the chaperone genes to decreased intracellular cAMP.

### Example 12

### Luminal filling

Luminal filling can lead to the release of some gastrointestinal polypeptides. For this reason, we investigated the role of luminal stimuli on the chaperone mRNA response. Fasted mice were refed a nonnutritive paste of cellulose (a normal component of their regular diet) and mineral oil. The mice initially consumed the mixture enthusiastically. Stomach filling was confirmed for each mouse by postmortem examination. Cellulose-mineral oil consumption produced a minor but significant increase in GRP78 mRNA (Fig. 6B), without producing a change in plasma glucose, insulin, or glucagon concentrations.

### Example 13

### Adrenal hormones

To investigate the role of adrenal hormones in the postprandial induction of GRP78 mRNA, we examined the effects of feeding in adrenalectomized mice (Fig. 7). Neither adrenalectomy nor sham surgery had any effect on the fasting levels of GRP78 mRNA. However, adrenalectomy increased the magnitude of the postprandial induction of the mRNA by approximately 2-fold over that found in refed, sham-operated mice. The feeding response of GRP94, ERp72, and GRP170 were also enhanced in the adrenalectomized mice (data not shown). Thus, the increase is a generalized ER chaperone response. Administration of dexamethasone to adrenalectomized mice increased the basal level of GRP78 mRNA during starvation, although not significantly (Fig. 7). However, dexamethasone administration had no effect on the feeding induction of the gene, suggesting its absence from adrenalectomized mice is not responsible for the enhancement of the feeding response.

### Example 14

### Preparation of test groups for short-term CR studies

Three groups of 30 month old mice were utilized for these studies. Male B6C3F, mice were maintained as described (Ohahbi et al. (1998) J. Gerontol 53A: B180). Mice were weaned at 28 days and housed individually. The composition of the defined diets used have been described. They are formulated so that only the amount of carbohydrate consumed varied between the CR and control mice. A group of control mice was fed a purified, semi-defined diet from 6 weeks of age. Control mice consumed approximately 105 kcal per week from weaning. This is approximately 10% less than the amount of food thought to support optimal growth, fertility and fecundity in mice {Subcommittee on Laboratory Animal Nutrition & Committee on Animal Nutrition 1978 ID: 5480}. Subjectively, these mice appeared neither fat nor lean. A group of calorically restricted mice (CR mice) were fed a diet reduced in dietary carbohydrate such that the mice consumed approximately 40% fewer calories than control mice. The long term CR mice consumed approximately 55 kcal per week from weaning. The short term CR mice were fed 105 kcal until the age of 29 months. They were then fed 80 kcal of control diet for 2 weeks, followed by 55 kcal of CR diet for two weeks. The mice were fed daily at 0900 hours. They had free access to water. For the studies, mice were fed a normal allotment of food Monday morning, and all the food was eaten within 45 minutes. They were fasted for 24 hours, and killed on Tuesday morning. At the time of use, the long term CR, short term CR and control mice weighed 22.8 ± 1.4, 25.2 ± 0.3 and 37.2 ± 2.4 g, respectively. The mice were approximately 30 months old when killed.

Mice were killed by cervical dislocation and the liver rapidly removed and flash frozen in liquid nitrogen. Approximately 0.2 g of frozen liver was homogenized for 40 s in 4 ml of TRI Reagent (Molecular Research Center, Inc., Cincinnati, OH) using a Tekmar Tissuemizer (Tekmar Co., Cincinnati, OH) at a setting of 55. RNA was isolated as described by the supplier.

GeneChip oligonucteotide-based high-density array RNA expression assays were performed-according to the standard Affymetrix protocol. The biotinylated, fragmented cRNA was hybridized to the Mu11KsubA and Mu11KsubB GeneChip arrays (Affymetrix, Santa Clara, CA), which contain targets for more than 11,000 known mouse genes and ESTs. The arrays were washed, stained and scanned. Scanned image analysis and data quantification were performed using the Affymetrix GeneChip analysis suite v3.2 at default parameter settings. Resultant data were normalized by global scaling, allowing comparisons between any two experiments.

The fold change analysis between all pairwise comparisons were performed by Affymetrix software. Since the average difference of a transcript is directly related to its expression level, an estimate of the fold change of the transcript between any two samples can be calculated. Fold change is an indication of the gene expression difference between any two given samples. To determine the effect of age, each 7-month-old mouse (n-3) was compared to each 30-month-old mouse (n=3) in the same dietary group (control, LT-CR), generating a total of nine pairwise comparisons. The average of the all nine possible pairwise comparisons determines the fold change for the effect of age. Only fold change greater or equal to 1.8 (or less than or equal to -1.8) were retained. To determine the effect of LT-CR, each control mouse (n-3) was compared to each LT-CR mice (n=3) in the same age group (young, old). The average of the all nine possible pairwise comparisons determined the fold change for the effect of diet. Only fold change greater or equal to 1.8 (or less than or equal to -1.8) were retained. To determine the effect of ST-CR at old age in 2 or 4 weeks, each old control mouse (n=3) was compared to each ST-CR mouse (n=3), and to each ST-CR mouse (n=3). The average of each nine possible pairwise comparisons determines the effect of ST·CR. in 2 or 4 weeks. Only fold change greater or equal to 1.8 (or less than or equal to -1.8) were retained.

### Example 15

### Effects of aging and calorie restriction on liver gene expression microarray profiles

### AGING EFFECTS

We conducted microarray analysis of the hepatic expression of more than 11,000 genes in young and old (7 and 30 months) mice subjected to long-term CR (LT-CR). Our study also included a group of old control mice that were subjected to a very short-term (2 or 4 weeks; ST-CR). We found that of the 11,000 genes monitored, only 48 (0.5%) showed expression levels that are changed with aging in the control mice.

1. 46% of the genes that were altered by age showed an increase in gene expression (Table 1).

Of the 48 genes that changed expression during aging (increased or decreased), the expression levels of 22 (46%) increased with age in control mice. These genes can be grouped into 6 classes (Table 1). The increase in expression of several members of the Stress Protein / Chaperone group (27%) indicates a marked stress response during aging in the livers of old control mice. The second group is composed of genes involved in inflammation processes. It represents 36% of all the genes for which expression increased with age. Key regulators of apoptosis also increased, suggesting that disregulatian of apoptosis is associated with aging.

**TABLE 1**

| GROUPS OF GENES WHICH INCREASED EXPRESSION WITH AGE (more than 1.8 fold). LONG AND SHORT TERM CR (LT-CR AND ST-CR) EITHER OPPOSD OR HAD NO EFFECT ON THIS INCREASE. ("Early decrease" means that CR decreased expression at young age. "Late decrease" means that CR at old age) | | | |
|---|---|---|---|
| GENE-BANK# | GENE¹ | LT-CR | ST-CR |
| | STRESS PROTEINS / CHAPERONES | | |
| J04633 | **(*e,f*) heat shock protein, 86 kDa 1 (Hsp86-1).** cytosolic molecular chaperone; one of the two HSP90 proteins in mice, 86 kDa and 84 kDa; | Early decrease -2.8 | Decrease -2.9 |
| D78645 | **(*e,f*) heat shock 70kD protein 5 (glucose-regulated protein, 78kD) (Hspa5)** also known as Grp78, BiP; molecular chaperone; | Early decrease -2.0, -2.3 | Decrease -2.1 |
| M73329 | **(*c,d*) endoplasmic reticulum protein (Erp)** was originally thought to be a phosphatidylinositol-4,5-bisphosphate phosphodiesterase type I (phospholipase C-alpha), but is a protein disulfide isomerase in ER; rearrangement of both intrachain & interchain disulfide bonds in proteins to form the native structures | Late decrease -1.9 | Decrease -2.1 |
| D67016 | **(*a*,*b*) heat shock protein, 105 kDa (Hsp 105)** two isoforms, Hsp105-alpha and Hsp105-beta, are produced by alternative splicing; induced by heat shock, Hsp 105-alpha also induced by other stresses; suggested to be not only chaperones that prevent thermal aggregation of proteins, but also regulators of the Hsc70 chaperone system | No effect | Decrease -2.3 |
| L07577 | **(*a,b*) heat shock protein, 25 kDa (Hsp25);** involved in stress resistance and actin organization | No effect | Decrease -2.3 |
| U27830 | **(*a,b*) stress-induced phosphoprotein 1 (Stip1)** a co-chaperone homologous with the human heat shock cognate protein 70 (hsc 70)/heat shock protein 90 (hsp90). organizing protein (Hop), which is an indispensable component of a dynamic heterocomplex chaperoning machine that is involved in the functional maturation of a number of unrelated protein substrates; the concomitant interaction of mSTI1 with hsp70 and hsp90 at its N- and C-termini respectively is mediated by the tetratricopeptide repeat (TPR) motifs in these regions | No effect | Decrease -2.4 |

| | **APOPTOSIS** | | |
|---|---|---|---|
| AF01826B | **(*e,f*) apoptosis inhibitory 6 (Api6);** also known as ACC-11; functions as a novel inhibitor of apoptosis; the same as Spalpha (Sp), a unique member of group B scavenger receptor cysteine-rich (SRCR) proteins which function as modulators in the immune response; highly expressed in the spleen, liver and, weakly in the lung, but not in heart, brain, skeletal muscle, kidney, nor testes; also expressed at lower levels in the thymus and lymph node | Early decrease -2.1, -5.0 | Decrease -1.9 |
| U44088 | **(*e*) T-cell death associated gene (Tdag);** plays an essential role in induction of apoptosis by coupling TCR stimulation to Fas expression in T cells | Early decrease -2.8, -7.4 | No Effect |
| U35623 | **(*a*) myeloid cell leukemia sequence 1 (Mcl1);** belongs to the Bcl-2 protein family, anti-apoptotic; the intracellular distribution of Mcl1 resembles that of Bcl2, both locate to the mitochondria, and both associate with membranes via carboxyl hydrophobic tails; the two differ in distribution in other compartments; the temporal expression of Mcl1 also overlaps with but differs from that of Bcl2 | No effect | No effect |

| | **INFLAMMATION RESPONSE** | | |
|---|---|---|---|
| L20276 | **(*c,d*) biglycan (Bgn);** also called small PG I. extracellular matrix structural protein; produced in the liver by non-parenchymal cells, increased expression in fibrotic liver, in areas of necroinflammation non-parenchymal cells transform into myofiboblasts which express biglycan gene, | Late decrease -1.8 | Decrease -2.0 |
| U88327 | **(*a*) cytokine inducible SH2-containing protein 2 (Cish2);** cytokine signal inhibitor (cytokines regulate fundamental biological processes such as immunity an wound healing) inhibits both cytokine-induced receptor phosphorylation and STAT activation; may act in a classic negative feedback loop to regulate cytokine signal transduction | No effect | No effect |
| U59807 | **(*c,d*) cystatin B (Cstb);** thiol (cysteine) protease inhibitor; expressed in many tissues, cystatins and their target enzymes play an important role in many pathological events such as inflammation, viral diseases, autoimmune diseases, and malignant progression of human and animal tumors. | Late decrease -2.5 | Decrease -2.6 |
| L20315 | **(*e*) macrophage expressed gene 1 (Mpeg 1);** found at a high level in mature murine macrophages and at a moderate level in certain myelomonocytic cell lines, not detected in a wide variety of other tissues and cell lines | Early decrease -2.2, -4.2 | No effect |
| M21050 | **(*c*) lysozyme (Lyzs);** carbohydrate metabolism, antibacterial response; expressed weakly in myeloblasts, moderately in immature macrophages, and strongly in both mature macrophages and mactophage-rich tissues | Late decrease -2.5 | No effect |
| M22531 | **(*c*) complement component 1, q subcomponent, beta polypeptide (C1qb);** belongs to the recognition set of the complement (C) system which is the primary humoral mediator of antigen-antibody reactions. Activation of the system results in a cascade of proteins which may attack invader cell membranes or activate specialized host cell functions. The C1 protein which initiates the classical pathway is a macromolecule, (C1 q.C 1 r2.C 1s2); C 1r and C 1 s are serine esterases which are activated by the binding of C1q to immune complexes; C1q itself is a complex of six chains of each of three types-, and C1qb is expressed at highest levels in peritoneal macrophages, with substantial levels in spleen, thymus, heart, and brain, only trace amounts in liver, kidney, lung and intestine tissues. | Late decrease -2.4 | No effect |
| K01925 | **(*c,d*) MHC class If H2.IA.alpha (q haplotype);** involved in the control of the immune response | Late decrease -2.6 | Decrease -2.6 |
| X16899 | **(*a*) Serum amyloid P-component (Sep);** acute phase reactant; a precursor of amyloid component P which is found in basement membrane and associated with amyloid deposits | No effect | No effect |

| | TRANSMEMBRANE CHANNELS I TRANSPORTER PROTEINS | | |
|---|---|---|---|
| M81445 | **(*e*) gap junction membrane channel protein beta 2 (Gjb2);** also called connexin 26; one gap junction consists of a cluster of closely packed pairs of transmembrane channels, the connexins, through which materials of low MW diffuse from one cell to a neighboring cell; a connexin is composed of an hexamer of connexins | Early decrease -1.8, -2.6 | No effect |
| Z48670 | **(*c,d*) ATP-binding cassette, sub-family D (ALD), member 2 (Abcd2);** small molecule transport; belongs to the superfamily of ATP binding cassette (ABC) transporters and has the structure of a half transporter; localized in the peroxisomal membrane; expressed in brain and adrenals, and weakly expressed in liver | Late decrease -2.0 | Decrease -2.1 |

| | **A. TRANSCRIPTION FACTORS** | | |
|---|---|---|---|
| AF006492 | **(*a,b*) zinc finger protein, multitype 1 (Zfpm1);** also called Friend of GATA-1 (FOG); acts as a cofactor for transcription factor GATA-1 in erythroid and megakaryocytic differentiation | No effect | Decrease 2.1 |
| U70139 | **(*c,d*) carbon catabolite repression 4 homolog (S. cerevisiae) (Ccr4);** evolutionarily conserved, with similarity to the yeast CCR4 transcription factor; specifically expressed in the liver of the aged mouse | Late decrease -2.0 | Decrease -2.1 |
| | **B. OTHERS** | | |
| J00361 | **(*c*) amylase 2, pancreatic (Amy2);** responsible for hydrolyzing 1,4 glycoside bonds, the primary enzyme responsible for the initial hydrolysis of alpha-linked glucose in the small intestinal lumen; produced principally in the pancreas | late decrease -3.4 | No effect |

| | | | |
|---|---|---|---|
| ¹The italicized letter in parenthesis preceding the gene name indicates the gene group designation from Fig. 1. | | | |

**2. 54% of the genes that changed during aging showed a decrease in gene expression (Table 2).**

Of the 48 genes that changed expression during aging, the expression of 26 (54%) decreased with age in control mice. One of the functional groups of genes that decreased involved cellular defense against adverse redox conditions (glutathione-S-transferase like) and the oxidative metabolism of xenobiotics (cytochrome P450; 1a2). The decrease observed in the expression of genes involved in DNA replication and the cell cycle suggests a decrease in hepatocyte proliferative capacity during aging. A number of major urinary proteins also were decreased during aging.

**TABLE 2**

| GROUPS OF GENES WHICH DECREASED EXPRESSION WITH AGE (more than 1.8 fold). LONG- AND SHORT-TERM CR EITHER OPPOSE OR HAVE NO EFFECT ON THE AGE-RELAETD DECREASE IN GENE EXPRESSION. ("Early increase" means that CR effects were manifested at young age, and "Late increase" at old age) | | | |
|---|---|---|---|
| GENE- BANK # | GENE¹ | LT-CR | ST-CR |
| | II. STRESS RESPONSE/ MOLECULAR CHAPERONES | | |
| U80819 | **(*a,b*) glutathione-S-transferase like (Gsttl-pending);** also named as p28; significant homologies to a large family of stress response proteins that contain a glutathione S-transferase (GST) domain; may be involved in the cellular defense against or in the adaptive response to altered cellular redox conditions; high expression in liver, lung, heart | Early Increase 1.8, 4.9 | Increase 5.1 |
| M77497 | **(*c,d*) cytochrome P450, 2f2 (Cyp2f2);** involved' in the regio- and stereoselective transformation of naphthalene to trans-1R-hydroxy-2r-glutathionyl-1,2-dihydronaphthaiene in the presence of glutathione and glutathione s-transferases; specifically catalyses the production of a very reactive and potentially toxic intermediate, the 2R,2S arene oxide, that is associated with necrosis of the unciliated bronchiolar epithelial cells or clara cells in lung | Late Increase 2.2 | Increase 6.9 |
| X00479 | **(e) cytochrome P450, 1a2, aromatic compound inducible (Cyp1a2);** a constitutively expressed hepatic enzyme that is primarily involved in oxidative metabolism of xenobiotics and is capable of metabolically activating numerous procarcinogens including aflatoxin B1, arylamines, heterocyclic amine food mutagens, and polycyclic aromatic hydrocarbons | *No Effect* | *No Effect* |
| M60358 | **(*e*) cytochrome P450, 2b13, phenobarbitol inducible,** type c (Cyp2b13); a female-specific testosterone-16α hydroxylase related but not equivalent to P450IIB9 or P450IIB10 | No Effect | No Effect |
| U12473 | **(*e,f*) conserved helix-loop-helix ubiquitous kinase (Chuk);** stress-responsive kinase receptor signaling protein; *serine*/*threonine* kinase; one of the catalytic subunits of I-kappaB kinase, the phosphorylation of I-kB allows NF-kB to translocate into the nucleus to immediately initiate gene transcription | No Effect | increase 2.3 |

| | **CELL CYCLE / DNA REPLICATION** | | |
|---|---|---|---|
| L31783 | **(e,*f*) uridine monophosphate kinase (Umpk);** the rate-limiting enzyme in the pyrimidine salvage pathway of all mammalian cells | No Effect | Decrease |
| U92437 | **(*e*,*f*) phosphatase and tensin homolog (Pten);** induces cell cycle arrest through inhibition of P13/Akt, potential tumor suppressor; active as a phosphatase on tyrosine, serine and threonine residues on proteins and on 3-phosphorylated phosphoinositides; at least part of its role is to regulate the activity of the serine/threonine kinase AKT/PKB, and thus influence cell survival signaling; it is shown to be an essential mediator of the Fas response and a repressor of autoimmunity and thus implicate the PI 3-kinase/Akt pathway in Fas-mediated apoptosis | No Effect | increase -2.0 |
| M16465 | **(*e,f*) calcium binding protein A11 (calgizzarin) (S100a10);** the light chain unit of Calpactin I which occurs as a tetramer of two light chain (P10) and two heavy chain polypeptides (P36, annexin II); because P10 induces the dimerization of annexin II, it may function as a regulator of protein phosphorylation in that the P36 monomer is the preferred target (in vitro) of tyrosine-specific kinase; calgizzarin is expressed in all adult tissues | No Effect | Increase 1.9 |
| X67493 | **(*e,f*) Insulin-like growth factor binding protein 1 (Igfbp1);** has been shown to act independently of its binding to IGFs; depending on its state of phosphorylation and the cell system involved, IGFBP-1 may either potentiate or inhibit IGF action; produced primarily in the liver and decidualized stromal cells of the endometrium; hepatic production is maximal during fetal life, decreases rapidly after birth, and thereafter is dependent on insulin secretion that inhibits its synthesis at the transcriptional level; the abundant endometrial expression of IGFBP-1 early in gestation suggests that it is involved in local control of cell proliferation; permanent and uncontrolled hepatic expression of IGFBP-1, even at low levels, affects fertility in females and both ante- and postnatal development; reduced body weight gain and brain weight as well as altered glucose homeostasis are also the features of IGFBP-1 transgenic models | No Effect | Increase 2.7 |
| M64292 | **(*c,d*) B-cell translocation gene 2, anti-proliferative (Btg2);** a family member of Btg gene family, homologous to the human BTG1, BTG2 and TOB genes which were demonstrated to act as inhibitors of cell proliferation; involved in the control of the cell cycle; the BTG proteins are found in organisms from nematodes to humans, and a large body of evidence suggests that Btg protein may be the mediator of multiple antiproliferative activities; Btg2 expression is also regulated by p53; expressed in most tissues | Late increase 2.6 | increase 2.8 |
| X73359 | **(*c*) related to Drosophila groucho gene (Grg);** (Drosophila Groucho (Gro) is one of the neurogenic genes that participates in the Notch signaling pathway; Gro protein is the prototype for a large family of corepressors); murine homologs Grg are believed to interact with transcription factors and repress transcription, thereby regulating cell proliferation and differentiation; also play a role in mediating the morphogenesis of CNS; little is known about the mechanisms in by which groucho family proteins function as eukaryotic corepressors in higher eukaryotes; Grg has been shown to act as a repressor for NF-kB in mammalian cells; mammalian Grg protein may play a pivotal role in biological processes by modulating transcriptional activity of NF-kB | Late Increase 7.4 | No Effect |
| M58691 | **(*c,d*) zinc finger protein 36 (Zfp36);** encodes the putative zinc finger protein tristetraprolin (TTP), a widely expressed protein containing two putative zinc fingers of the unusual CCCH class; Although TTP has no proven function, disruption of its gene, Zfp-36, in mice leads to a complex syndrome that includes erosive arthritis, conjunctivitis, myeloid hyperplasia, cachexia, and autoimmunity; it has been suggested that a potential function of TTP is to regulate the production of TNFalpha by certain cell types. The transcription of Zfp-36 is rapidly and dramatically stimulated by a variety of growth factors and mitogens, but not by agents acting solely through increases in cAMP levels | Late Increase 1.9 | Increase 2. |

| | **KINASES / PHOSPHATASE** | | |
|---|---|---|---|
| AF033565 | **(*c*) CDC-like kinase 3 (Clk3);** belongs to the family of LAMMER kinases (i.e. kinases carrying the conserved signature EHLAMMERILG in the catalytic domain); phosphorylates serines, threonines and tyrosines; phosphorylates serine- and arginine-rich (SR) proteins of the spliceosomal complex, may be a constituent of a network of regulatory mechanisms that enable SR proteins to control RNA splicing. | Late Increase 3.2 | No Effect |
| X61940 | **(c) protein tyrosine phosphatase,** non-receptor type 16 (Ptpn16); also known as erp, MAP kinase phosphatase I (MKP-1); encodes a nontransmembrane tyrosine phosphatase protein (Class I protein tyrosine phosphatase); has been reported to be expressed in a wide range of tissues and cells; exemplifies a class of dual-specificity phosphatase able to reverse the activation of mitogen-activated protein (MAP) kinase family members by dephosphorylating critical tyrosine and threonine residues; a key element in controlling cellular signaling pathways activated by MAP kinases; MKP-1 acts as a negative regulator of insulin signaling. | Late Increase 2.8 | No Effect |

| | **MAJOR URINARY PROTEINS** | | |
|---|---|---|---|
| X03208 | **(*c,d*) major urinary protein 1 (Mup1);** lipocalin produced in the liver or in the lachrymal, mammary, and submaxillary glands, but secreted in the urine; binds pheromones, which are released from drying urine of males and affect the sexual behavior of females | Late Increase 1.9 | Increase 3.2 |
| M16359 | **(*c,d*) major urinary protein 3 (Mup3);** synthesized in the liver under the influence of a number of hormones, including thyroxine, growth hormone, and testosterone; also expressed in the submaxillary, sublingual, lachrymal glands; and secreted into the serum and excreted in the urine; MUPs belong to the lipocalin superfamily and share with other members of this family the capacity to bind hydrophobic molecules, some of which are odorants; transport of pheromones | Late Increase 2.4 | Increase 3.3 |
| M16358 | **(*c*,*d*) major urinary protein 4 (Mup4);** same as Mup3 I | Late ncrease 1.9 | Increase 2.4 |
| M16360 | **(*c*,*d*) major urinary protein 5 (Mup5);** same as Mup3 | Late Increase 2.4 | Increase 4.6 |

| | **METABOLISM** | | |
|---|---|---|---|
| AF026073 | **(*a,b*) amine N-sulfotransferase (SULT-N);** catalyzes sulfuryl group transfer from 3'-phosphoadenosine 5' phosphosulfate to one of a large number of either primary or secondary amines forming the appropriate sulfamate and adenosine 3',5'-bisphosphate; liver | Early Increase 2.0, 2.3 | Increase 3.6 |
| L07645 | **(*a,b*) histidine ammonia lyase (Hal):** catalyzes the first step in the major mammalian catabolic pathway for histidine. | Early Increase 4.4 | Increase 5.5 |
| D00466 | **(*e*,*f*) apolipoprotein E (Apoe);** transports cholesterol and triglycerides throughout the body; mediates binding, internalization, and catabolism of lipoprotein particles; can serve as a ligand for the LDL (Apo B/E) receptor and for the specific Apo-E receptor (chylomicron remnant) of hepatic tissues | No Effect | Decrease -2.1 |
| M27796 | **(*e,f*) carbonic anhydrase 3 (Car3);** catalyzes the reversible reaction of carbon dioxide and water to form carbonic acid; also important in ion transport, acid/base homeostasis, secretion and absorption; Car3 is unique among the carbonic anhydrases since it also possesses phosphatase activity; Car3 has recently been suggested to prevent from H2O2-inducible apoptosis and to be one of the cytosolic protein tyrosine phosphatases (PTPs) involved in **OTHERS** | No Effect | Decrease |
| U02554 | **(*e*) serum amyloid A 4 (Saa4);** used to be called. Saa-5, what was Saa-4 is a pseudogene which is now called Saa-ps; major acute phase reactant; apolipoprotein of the HDL complex | No Effect | No Effect |
| D50032 | **(*e,f*) trans-golgl network protein 2 (Ttgn2);** an integral membrane protein that is predominantly localized to the trans-Golgi network (TGN); may be involved in regulating membrane traffic to and from trans-golgi network | No Effect | Increase |
| ET61200 | **(*e*) complement receptor related protein (Crry);** normally found as membrane proteins; regulates the deposition of activated C3 and C4 components of complement on the surface of autologous cells in vitro by exhibiting MCP- and DAF-like activities, although its relative contribution to complement regulation as compared to mouse MCP and DAF has not been elucidated; (Decay accelerating factor (DAF) inactivates the C3 convertase enzymes that activate C3. Membrane cofactor protein (MCP) serves as a cofactor for factor I-mediated degradation of activated C3 and C4.) | No Effect | No Effect |
| U75530 | **(*e*) eukaryotic translation initiation factor 4E binding protein 2 (Eif4ebp2);** also called PHAS-11; binds to the initiation factor 4E and inhibits translation of capped mRNA; the phosphorylation of eiF4EBP1 and eiF4EBP2 by insulin and by growth factors releases their binding to eiF-4E and increases activity of eiF-4E. | No effect | No effect |

| | | | |
|---|---|---|---|
| ¹The italicized letter in parenthesis preceding the gene name indicates the gene group designation from Fig. 2. | | | |

### Example 16

### CR Effects

LT-CR and ST-CR had diverse effects on the 48 genes which changed expression with age.

### 1. LT-CR opposed the effects of aging on the expression of 58% (28 of 48) of the genes that changed expression with age

For 28 out of 48 (58%) genes that increased or decreased with age, LT-CR opposed the change (Figs. 8 and 9). Of these 28 genes, 15 are genes that were decreased by LT-CR (Fig. 1) and 13 are genes that were increased by LT-CR (Fig. 2). Because these gene respond to both age and CR, there expression may be linked to factors which are determined by physiological age, not chronological age. Genes that are regulated by mechanisms related to physiological age are linked to life span and may serve as biomarkers of aging.

ST-CR reproduced the effects of LT-CR on 19 of the 28 genes (68%) for which the age-related alterations were opposed by LT-CR. In this set of 19 genes, where the patterns of gene expression were highly homologous in the ST- and LT-CR groups, CR does not maintain a youthful gene expression profile, but rapidly induces a "slow-aging" profile. CR rapidly altered genes associated with stress response, inflammation, and the division and apoptosis of cells. Thus, expression profiling of these genes should prove useful in rapidly identifying CR mimetic drugs and treatments.

### 2. LT-CR did not affect the expression of 42% (20 of 48) of the genes that changed expression with age

For the remaining 20 of the 48 genes (42%) that increased or decreased with age, gene expression was not altered by LT-CR (Figs. 8 and 9). Of these 20 genes. 7 increased with age but were not affected by LT-CR (Fig. 8), and 13 decreased with age but also were not affected by LT-CR (Fig.9). These results suggest that the expression levels of these genes, being independent of CR, do not depend on the rate of biological aging, but on the passage of time (chronological age).

ST-CR actually influenced the expression of 12 of the 20 genes which seem to be responsive only to chronological age: More than half the 12 genes that respond to ST-CR are molecular chaperones and stress response proteins, or genes involved in DNA replication. Both of these groups are known to be nutritionally regulated and rapidly respond to metabolic alterations.

### 3. The expression of 36 genes was unchanged during aging but was responsive to ST-CR and/or LT-CR (Table 3)

As reported above, only 0.50% of the 11,000 screened genes were differentially expressed during aging in the liver of control mice. Thus, expression of the vast majority of the genes remain unchanged during aging. However, LT-CR differentially altered the expression of 36 of these genes in old and young mice (Fig. 10). The 36 genes include those for key metabolic enzymes, stress response proteins, and genes involved in immune and inflammatory responses. The age-independent effects of CR on these diverse groups of genes may be involved in mediating the life- and health-span extending effects of LT-CR.

ST-CR reproduced the effects of LT-CR on 14 of the 36 (39%) genes (Fig. 10). Thus, 39% of these genes mimic the effects of LT-CR after only 4 weeks of ST-CR. These results indicate that a significant portion of the genetic reprogramming induced by LT-CR in this class of genes is reproduced rapidly.

**TABLE 3**

| GROUPS OF GENES FOR WHICH EXPRESSION WAS UNCHANGED BY AGING (less than 1.8 fold). LONG- AND SHORT-TERM CR EITHER INCREASE OR DECREASE THEIR GENE EXPRESSION. ("Early increase or decrease" means that CR effects were manifested at young age, and "Late increase or decrease" at old age) | | | |
|---|---|---|---|
| **GENE- BANK #** | **GENE¹** | **LT-CR** | **ST-CR** |
| | **STRESS RESPONSE/CHAPERONES** | | |
| J05186 | **(*g*) Calcium binding protein, Intestinal (Cal);** also known as ERp72; a stress protein and a member of the protein disulfide isomerase family of proteins | Early Decrease -1.8, -2.2 | No Effect |
| U28423 | **(*e,f*) Protein kinase, interferon Inducible double stranded RNA dependent Inhibitor (Prkri);** also called protein kinase inhibitor p58; present in all tissues examine so far in both mice and humans, with especially high levels in the liver and pancreas, P58 IPK a tetratricopeptide repeat- containing co-chaperone that is involved in stress-activated cellular path-ways and that inhibits the activity jot protein kinase PKR, a primary mediator of the antiviral and antiproliferative properties of interferon. | Late Decrease -1.9 | Decrease -2.0 |

| | **METABOLISM / BIOSYNTHESIS** | | |
|---|---|---|---|
| X13752 | **(*g,h*) Delta-aminolevulinate dehydratase (Lv);** regulates the porphyrin biosynthesis in the heme biosynthetic pathway; The gene contains two first exons, named 1A and 1B, which are alternatively spliced to exon 2, where the coding region begins; mRNA containing exon 1A is ubiquitous, whereas mRNA containing exon 1 B is found only in erythroid tissues. | Early Decrease | Decrease -4.5 |
| U49861 | **(*g,h*) Deiodinase, iodothyronine, type I (Dio1);** enzyme that metabolize thyroxine (T4) to 3,5,3'- triiodothyronine (T3) to achieve full biological activity; expressed in intestine, liver and kidney | Early Decrease -2.1,-5.1 | Decrease -5.5 |
| X13135 | **(*g*) Fatty acid synthase (Fasn);** plays a central role in de novo lipogenesis in mammals; FAS concentration is exquisitely sensitive to nutritional, hormonal, and developmental status in liver and adipose tissues. | Early Decrease -2.7, -2.0 | No Effect |
| AB008895 | **(*e,f*) Glycosylphosphatldylinositol 1 homolog (human) (Gpi1h);** GPI1 is one of the four proteins participating in the GIcNAc transferase enzyme complex, the first step of glycosylphosphatidylinositol (GPI) biosynthesis; GPI represents an important anchoring molecule for cell surface proteins | Late Decrease -2.2 | Decrease -2.0 |
| U67611 | **(e) Transaldolase 1 (Taldo1);** important for the balance of metabolites in the pentose-phosphate pathway, the key enzyme of the oxidative pentose phosphate pathway, which is responsible for generation of reducing equivalents to protect cellular Integrity from reactive oxygen Intermediates; It catalyses the reversible transformation of sedoheptulose 7-phosphate and glyceraldehyde 3- phosphate to erythrose 4-phosphate and fructose 6-phosphate. The cycle supplies NADPH and precursor molecules for several biosynthetic reactions. | Late Decrease -2.0 | No Effect |
| AF010499 | **(*e*) Guanidinoacetate methyltransferase (Gamt);** catalyses the synthesis of creatine from guanidinoacetate and S-adenosylmethionine, the last step of creatine biosynthesis, expressed ubiquitously and at high levels in the liver | Late Decrease -2.3 | No Effect |
| M76727 | **(*e*) Pyruvate dehydrogenase E1alpha subunit (Pdha1);** pyruvate dehydrogenase catalyzes the conversion of pyruvate to acetyl-CoA and entry into the citric acid cycle; | Late Decrease -2.0 | No Effect |
| J02623 | **(*a*) Glutamate oxaloacetate transamlnase 1, soluble (Got1);** cytosolic aspartate aminotransferase (AST); a growing body of Information suggests that determination of AST isoenzymes in human serum is useful in evaluating damage to some of these organs; in hepatic disease, the test is used to assess liver necrosis and for determining prognosis | Early Increase 4.5, 2.3 | No Effect |
| L07037 | **(*c*) Mannoside acetytgtucosammyltransferase 1 (Mgat1);** initiates complex N-linked carbohydrate formation, essential for the conversion of high-mannose to hybrid and complex N- glycans; | Late Increase 4.6 | No Effect |

| | **INTRACELLULAR SIGNALING** | | |
|---|---|---|---|
| M33324 | **(*g*) Growth hormone receptor (Ghr);** decreases in growth hormone receptor signal transduction contribute to the decline in insulin-like growth factor I gene expression with age; expressed ubiquitously | Early Decrease -1.8, -2.9 | No Effect |
| ET61316 | **(*e,f*) Regucalcin (Rgn);**a calcium binding protein also known as senescence marker protein-30 (SMP30); high expression in liver and kidney cortex; plays a multiple role in liver. 1) plays a role in the maintenance of Intracellular Ca2+ homeostasis due to activating Ca2+ pump enzymes in the plasma membrane and microsomes of liver and renal cortex cells; 2) has an inhibitory effect on the activation of Ca2+/calmodulin-dependent enzymes and PKC; 3) regulates nuclear function In liver cells: it can inhibit Ca(2+)-activated DNA fragmentation, DNA and RNA synthesis, protein kinase and protein phosphatase activities in the nuclei; however, SMP30 has been reported to decrease with aging in the mouse and rat liver | Late Decrease -2.3 | Decrease -2.2 |
| X61432 | **(*e*,*f*) Calmodulin (Calm);** as the δ regulatory subunit of the enzyme phosphorylase kinase, mediates the calcium requirement of the enzyme; acts as an activator molecule for several molecules in addition to PHK; regulated by glucocorticoids in mouse thymocytes; expressed in various tissues | Late Decrease -2.7 | Decrease -2.7 |
| X70533 | **(*e*,*f*) Corticosteroid binding globulin (Cbg);** a member of the serine proteinase inhibitor superfamily; and is responsible for the plasma transport of glucocorticoids; regulates the action of glucocorticoids which influence fetal development; GR is essential for maintaining the basal level of CBG gene expression in the liver, | Late Decrease -2.7 | Decrease -9.3 |
| L75822 | **(*e*) Insulin-like growth factor binding protein 7 (Igfbp7);** also called Mac25; a retinoic acid-inducible, follistatin-like protein; believed to be capable of modulating growth by binding to IGFs, modifying their interaction with the cognate receptor, and thereby inhibiting the mitogenic activity; Igfbp-7 has been suggested to act as a growth suppressor by modulating signaling by the TGFbeta family. | Late Decrease -2.2 | No Effect |

| | **TRANSPORTERS** | | |
|---|---|---|---|
| U38652 | **(*g,h*) Solute carrier family 22, member 1 (Sic22a1);** a polyspecific transmembrane organic cation transporter; could also play Important developmental roles independent of transport function; expressed at high levels in mouse liver, kidney, and intestine, and at low levels in the adrenals and In lactating mammary glands. | Early Decrease -1.8, -2.9 | Decrease -5.0 |
| M65034 | ***(g,h)* Fatty acid binding protein 2, Intestinal (Fabp2);** intracellular binding and transport of fatty acid, modulation of specific enzymes of lipid metabolic pathway. | Early Decrease -3.1 -5.4 | Decrease -4.5 |
| AF028739 | **(*a*) Solute carrier family 22 (organic cation transporter), member 1-like (Sic22a1l);** organic transport mechanisms for the elimination of endogenous and exogenous toxins are necessary for the survival of mammalian species; Slc22a1l plays a role in transport of organic molecules, perhaps including those with morphogenetic activity; may also play important developmental roles Independent of transport function | Early Increase 1.9, 2.0 | No Effect |
| U30840 | **(*c*,*d*) Voltage-dependent anion channel 1 (Vdac1);** also known as mitochondrial porin found in the outer mitochondrial membrane of all eukaryotes. VDACs are the binding sites for several cytosolic enzymes, Including the isoforms of hexokinase and glycerol kinase. VCACs are small, integral membrane proteins that traverse the outer mitochondrial membrane and conduct ATP and other small metabolites; VDAC1 is the prototypic version whose properties are highly conserved among other species; are known to bind several kinases of intermediary metabolism in a tissue-specific fashion, have been found in close association with the adenine nucleotide translocator of the inner mitochondrial membrane, and are hypothesized to form part of the mitochondrial permeability transition pore, which results in the release of cytochrome c at the onset of apoptotic cell death. | Late Increase 3.6 | Increase 3.3 |

| | **TRANSCRIPTION FACTORS** | | |
|---|---|---|---|
| AF027963 | **(*g*) X-box binding protein 1 (Xbp1);** a CREB/ATF family transcription factor highly expressed in hepatocellular carcinomas; a transcription factor essential for hepatocyte growth; also is induced by ER stress and that induction of XBP-1 is mediated by cis-acting ER stress response element; | Early Decrease -1.8, -2.2 | No Effect |
| Z47088 | **(*g*) Transcription elongation factor B (SIII), polypeptide 1 (15 kDa),-like (Tceb1l);** possibly an RNA polymerase II elongation factor; encodes a 163-amino-acid protein and shows a significant level of identity with a RNA polymerase II transcription elongation factor, p15; | Early Decrease -1.9, -2.2 | No Effect |
| U20086 | **(*a,b*) Upstream binding protein 1 (Ubp1);** also known as nuclear binding factor NF2d9; belongs to the NTF-like family of transcription factors; NF2d9 is responsible for governing the transcription of P450 genes | Early Increase 2.7,1.8 | Increase 1.9 |
| U14666 | **(*a*) Nuclear receptor subfamily 6, group A, member 1 (Nr6a1);** also known as RTR; a novel orphan receptor In the nuclear receptor superfamily of ligand-activated transcription factors, is expressed predominantly in developing germ cells; plays an important role in the control of gene expression during early embryonic development and gametogenesis; germ cell-specific | Early Increase 1.9, 2.1 | No Effect |

| | **IMMUNE RESPONSE /INFLAMMATION** | | |
|---|---|---|---|
| M17122 | **(*g*) Complement component 4 binding protein (C4bp);** binding protein for the C4b molecule in the complement system cascade; involved in regulation of the classical pathway; liver-specific | Early Decrease -1.8,-2.4 | No Effect |
| U04268 | **(*g*) Lymphocyte antigen 6 complex, locus E (Ly6e);** small cysteine-rich cell surface protein anchored in the membrane by a glycosyl-phosphatidylinositol moiety; defense (immune) response; is thought to play an important role in thymic T cell development; expressed at various levels in all organs examined | Early Decrease -2.5, -6.6 | No Effect |
| D64160 | **(*a*) Retinotic acid early transcript 1, alpha (Raet1a);** major histocompatibility complex antigen GPI-anchored cell surface proteins that function as ligands for the mouse activating NKG2D receptor; show weak homology with MHC class I; expression is low or absent on normal, adult tissues; they are expressed on some tumors and upregulated by retinoic acid; morphogenesis | Early Increase 2.1, 2.7 | No Effect |

| | **APOPTOSIS** | | |
|---|---|---|---|
| Y13231 | **(*c*) Bcl2 homologous antagonist/killer (Bak);** pro-apoptotic; in the presence of an appropriate stimulus, accelerates programmed cell death by binding to, and antagonizing the a repressor Bcl-2. or its adenovirus homolog E1B 19k'proteln (by similarity) | Late Increase 2.7 | No Effect |
| AF011644 | **(*c,d*) Doc-1;** a putative tumor suppressor gene isolated and identified from the hamster oral cancer model.; suppresses DNA replication in vitro; keratinocytes, | Late Increase 2.3 | increase 2.6 |

| | **DETOXIFICATION** | | |
|---|---|---|---|
| L11333 | **(*e*) Esterase 31 (Es31);** also called Es-male; liver carboxylesterase, involved in the detoxification of xenobiotics and in the activation of ester and amide prodrugs | Late Decrease -2.8 | No Effect |
| Y11638 | **(*c*) Cytochrome P450, 4a14 (Cyp4a14);** fatty acid oxidation; the CYP4A family is also induced by peroxisome proliferators; liver | Late Increase 2.2 | No Effect |
| X63023 | **(*c,d*) Cytochrome P450, steroid Inducible 3a13 (Cyp3a13);** | Late Increase 2.1 | Increase 4.7 |

| | **OTHERS** | | |
|---|---|---|---|
| J02870 | (***c*) P40-8, functional (P40-8);** structural protein of ribosome | Late Increase 1.9 | No Effect |
| L04961 | **(*c*) Inactive X specific transcripts (Xist);** may have a role in chromosome x inactivation | Late Increase 1.8 | No Effect |
| M13966 | **(*g,h*) Apolipoprotein A-IV (Apoa4);** functions in conjunction with other apolipoproteins to form lipoprotein particles which are involved in lipid homeostasis; normally synthesized in liver and in small intestine in rodents (is secreted only by the small intestine in humans); however, the small intestine is the major organ responsible for the circulating apo A-IV; Recent findings indicate that AopA-IV likely plays a role in the short-term regulation of food intake, and may also be involved in the long-term regulation of food Intake and body weight. | Early Decrease -1.9, -3.2 | Decrease -4.8 |
| X70303 | **(*g*) Proteasome (prosome, macropain) subunit, alpha type 2 (Psma2);** one of the alpha subunits of the 20S catalytic core of proteasomes which are multicatalytic complexes of endopeptidases which act in the degradation of damaged proteins and in the ATP-dependent pathway of proteolysis | Early Decrease -2.2, -2.5 | No Effect |

| | | | |
|---|---|---|---|
| ¹The italicized letter in parenthesis preceding the gene name indicates the gene group designation from Fig. 3. | | | |

### 4. ST-CR Reproduced the majority of the effects of LT-CR on Gene Expression

Of the genes which are commonly thought to be most important in the effects of CR, those that change expression with age, ST-CR reproduced the effects of 31 of the 48 changes induced by LT-CR (65%). Thus, in this important class of genes, ST-CR rapidly reproduced the effects of LT-CR.

Of all the genes, including those that did not change expression with age, 45 of 84 (54%) of the genes were responsive to both LT-CR and ST-CR in the same way and to the same degree. These results indicate that the majority of the effects which might be attributed to the long term effects of diet on changes in gene expression are rapidly reproduced by very short-term CR.

### 5. ST-CR altered the expression of 60 genes that did not change with LT-CR or aging.

Of the 60 genes that responded only to ST-CR, 33 were increased (Table 4) and 27 were decreased (Table 5). These genes encode stress proteins, metabolic enzymes, and cell growth mediators. Each of these classes of genes are known to rapidly respond to caloric intake. The liver plays a critical role in the adaptive response to environmental stimuli that alter energy requirements of the organism as a whole. This hepatic response to ST-CR may involve the modulation of diverse liver functions required for adaptation to the newly decreased nutritional intake.

Although these genes may not be important in the extension of life-span by CR, they may be crucial to the successful physiological transition from one feeding status (control) to the calorically restricted status, a transition which appears to include a stress response in the liver.

**TABLE 4**

| GROUPS OF GENES WHICH INCREASED EXPRESSION WITH ST-CR (more than 1.8 fold). AGING AND LT-CR HAD NO EFFECT ON THE EXPRESSION OF THESE GENES. | | |
|---|---|---|
| **GENE- BANK #** | **GENE** | **ST-CR** |
| | STRESS PROTEINS / DETOXIFICATION ENZYMES | |
| L02331 | **Sulfotransferase family 1A, phenol-preferring, member 1 (Sult1a1);** sulfo-conjugates various endogenous and exogenous compounds such as biogenic amines, phenols and steroid hormones, bile acids, drugs, and carcinogens. Transfer of sulfonate groups from phosphoadenosine-phosphosulfate to acceptor compounds by sulfotransferase enzymes is a step in the detoxification and clearance of these compounds | Increase 2.1 |
| AF003695 | Hypoxia inducible factor 1, alpha subunit (Hif1a); a transcription factor responsible for a coordinated genetic program mediating such diverse but related functions as increased respiratory rate, Increased erythropoiesis, increased glycolysis and increased angiogenesis all in response to organismal hypoxic stress; senescent organisms respond poorly to hypoxic stress and aging is associated with an decline in HIF-1 production and function; | Increase 2.1 |
| D30687 | Glutathione S-transferase, pi 2 (Gstp2); plays an important role in the detoxification of chemical toxins and mutagens and are implicated in neoplastic development and drug resistance | Increase 2.5 |
| U36993 | Cytochrome P450, 7b1 (Cyp7b1); most closely resembles P450VIIA1, the liver-specific cholesterol 7α- hydroxylase, but clearly differs from that enzyme; suggested to be a 7α-hydroxylase participating in the synthesis, in brain, of neurosteroids 7α-hydroxy dehydroepiandrosterone (DHEA), and 7α-hydroxy pregnenolone; expressed principally in brain, with only low levels found in liver | Increase 6.5 |

| | CATABOLISM / PROTEIN DEGRADATION | |
|---|---|---|
| U24493 | Mouse tryptophan-2,3-dioxygenase (TDO); the rate-limiting enzyme in the oxidative breakdown of tryptophan to kynurenine in the body. It's activity critically determines the relative tryptophan flux into the serotonergic (5-HT) and kynurenine (NAD, NADP poly ADPribose) pathways; expressed in the liver, responds to starvation | increase 2.1 |
| X51942 | **Phenylalanine hydroxylase (Pah);** catalyses the first and rate-limiting step in intracellular phenylalanine catabolism in mammals, expressed only in liver and kidney with some 20% of the total activity being associated with the latter organ. The activities of rat hepatic phenylalanine hydroxylase can be stimulated in vivo in response to conditions favoring gluconeogenesis, e.g. upon feeding a high protein diet, streptozotocin-induced diabetes or starvation | Increase 2.1 |
| AF003346 | **Ubiquitin-conjugatlng enzyme 4 (Ubce4);** ubiquitin-dependent protein degradation; the first step in protein-ubiquitin conjugation Is activation of the ubiquitin to a high energy intermediate form, catalyzed by the ubiquitin-activating enzymes (UBEIs). It is then donated to one of the UBE2 conjugating enzymes as a carrier protein, whence it can be attached to a target protein for alteration or degradation using the UBE3 ligase enzymes; ubce4 is a family member of UBE2; The ubiquitin- and ATP-dependent proteolytic pathway (UPP) has been associated with the degradation of abnormal and/or damaged proteins. Upon fasting, the ubiquitin-dependent proteolytic system Is activated In skeletal muscle in parallel with the increases in rates of proteolysis | Increase 2.5 |

| | CELL GROWTH/APOPTOSIS | |
|---|---|---|
| X07699 | **Nucleolin (Ncl);** regulates cell growth by regulation of ribosome biogenesis and maturation; functions as a cell surface receptor, where it acts as a shuttling protein between cytoplasm and nucleus | Increase 2.4 |
| U10420 | **N-methylpurine-DNA glycosylase (Mpg);** DNA repair; a glycosylase which can release 3-methyladenine, 7- methylguanine, or 3-methylguanine from alkylated DNA, and thus prevent alkylation induced cell death | Increase 2.1 |
| J03168 | Interferon regulatory factor 2 (Irf2); one gene in family of transcription factors involved in regulation of cell growth and immunological responses; negative regulator of Cox-2 gene (one of the rate-limiting enzymes that Initiates the conversion of arachidonic acid to prostanolds) via the type I interferon (IFN) system; required for normal generation of T helper type 1 responses and for NK cell development in vivo; IRF-2-deficient mice exhibited bone marrow suppression of haematopoiesis and B lymphopoiesis and mortality following lymphocytic choriomeningitis virus infection. | Increase 2.6 |
| U53586 | **Ecotropic viral integration site 5 (Evi5);** all information is about virus integration in this site; this gene has homology to mammalian cell cycle regulatory proteins | Increase 2.4 |
| X05475 | **Complement component 9 (C9);** involved in Complement activation (amplifies antibody action to fight bacteria); involved In apoptosis; regulates inflammatory response | Increase 4.1 |
| M21828 | **Growth arrest specific 2 (Gas2);** cell cycle arrest; might be involved in apoptosis; might play role in regulating chondrocyte proliferation and differentiation | Increase 2.3 |
| X81582 | Insulin-like growth factor binding protein 4 (lgfbp4); may act to distribute the IGFs among the body fluid compartments, to protect the body from possible hypoglycemic effects of the IGFs, and to modulate binding of IGFs to their receptors to mediate IGF action | Increase 2.3 |
| U84411 | **Protein tyrosine phosphatase 4a1 (Ptp4a1);** important in normal cellular growth control; expressed throughout the course of hepatic regeneration | Increase 2.0 |
| X70067 | **Ribonucleic acid binding protein S1 (Rnps1);** S phase prevalent RNA and DNA-binding protein; little is known about this gene | Increase 4.3 |
| L10244 | **Spermidine/spermine N1-acetyl transferase (Sat);** the rate-limiting enzyme in the interconversion of the polyamines, induction of SAT facilities the degradation of spermine and spermidine to putrescine an important molecule for cell growth; Multiple lines of evidence implicate the polyamines, putrescine, spermidine, and spermine in the lung injury and hypertensive pulmonary vascular disease; Sat is increased from 3 to 36 months of age | Increase 4.1 |
| U00674 | **Syndecan 2 (Sdc2);** also called fibroglycan, The syndecans are a family of four cell surface heparan sulfate proteoglycans in vertebrates that mediate a variety of cell behaviors, including cell adhesion and the action of growth factors. Their core proteins contain conserved transmembrane and cytoplasmic domains but divergent extracellular regions in which only the glycosaminoglycan attachment sites are conserved. syndecan 2 is a member of a family of cell surface heparan sulfate proteoglycans that interact with adhesion molecules, growth factors and a variety of other effector systems that support the shaping, maintenance and repair of an organism. It also participates selectively in the induction of stress fiber formation in cooperation with integrin alpha5beta1 through specific binding of its heparan sulfate side chains to the fibronectin substrate. | Increase 2.1 |

| | TRANSCRIPTION REGULATION | |
|---|---|---|
| L03279 | **Core binding factor beta (cbfb);** is considered to be a transcriptional coactivator that dimerizes with transcription factors core binding factor alpha-1 (CBFA1), -2, and -3, and enhances DNA binding capacity of these transcription factors | increase 2.0 |
| M62867 | **Y box protein 1 (ybx1); transcription regulation;** CCAAT-binding protein; an integral component of a eukaryotic redox signaling pathway | Increase 2.4 |
| M33212 | **Nucleophosmin 1 (Npm1);** major nucleolar protein; over-expression of the B23 protein can reverse the transcriptional repression exerted by YY1 | Increase 2.1 |
| M74570 | **Alcohol dehydrogenase family 1, subfamily A2 (Aldh1a2);** a cytoplasmic aldehyde dehydrogenase (AHD) isozyme, AHD-2 (also called ALDH1 or RALDH1); in addition to the activity on acetaldehyde and related substrates, is also involved in the oxidation of aldehydes derived from biogenic amines such as epinephrine and norepinephrine, as well as the aldehydes generated via lipid peroxidation; binds free retinal and cellular retinol-binding protein-bound retinal; functions as a retinal dehydrogenase in the pathway for conversion of retinol to retinoic acid, which then binds a nuclear retinoic acid receptor and transcriptionally regulates genes involved in several biological processes | Increase 2.0 |

| | INFLAMMATION / IMMUNE RESPONSE | |
|---|---|---|
| X70393 | **Inter-alpha trypsin Inhibitor, heavy chain 3 (Itih3);** Inter-alpha trypsin inhibitor is a group of structurally related plasma serine protease inhibitors. The ITI family members consist of combinations of mature heavy chains named HC1, HC2, HC3 linked to bikunin (a Kunltz-type protease inhibitor) by a covalent Interchain protein-glycosaminoglycan-protein cross-link. The biosynthesis of the ITI family members takes place in the liver, itih3 is believed to be involved in the inflammation-associated regulation, is likely to be a important partner of the acute phase response | Increase 2.3 |
| M64086 | **Serine protease Inhibitor 2-2 (Spi2-2);** a positive acute phase reactant, not GH responsive; facilitating modeling of regulation of hepatic acute phase response (APR). | Increase 2.7 |
| Z22593 | **Fibrillarin (Fb1);** plays a role in maturation of pre-rRNA, and in genesis and assembly of ribosomes; human fibrillarin expressed in vitro migrates on SDS gels as a 36-kDa protein that is specifically immunoprecipitated by antisera from humans with scleroderma autoimmune disease | Increase 2.1 |

| | OTHERS | |
|---|---|---|
| L25885 | **UDP-N-acetyl-alpha-D-galactosamlrie:(N-acetylneuramlnyl)-galactosylglucosylceramide-beta-1, 4-N-acetylgatactosaminyltransferase (Galgt1);** species specific, not expressed all mouse strains; most abundant in muscle (less in liver); ganglioside synthesis; gangliosides may be important for altering the electrical field across the membrane and the concentrations of surface ions (especially Ca+) | Increase 2.1 |
| X60289 | **Ribosomal protein S24 (Pps24);** ribosome biogenesis; structural protein of ribosome | Increase 2.0 |
| L29441 | **Tumor differentially expressed 1 (Tde1);** mRNA expression increased in transgenics males developed testicular tumor and seminal vesicle engorgement at advanced ages; contains several hydrophobic alpha-helices characteristic of transmembrane proteins; | Increase 2.7 |
| AF017153 | **DEAD/H (Asp-Glu-Ala-Asp/Hls) box polypeptide 15 (RNA helicase A) (Ddx15);** pre-mRNA processing; encodes a novel member of the DEAH box family of putative RNA helicases and RNA-dependent NTPases; a splicing factor that functions late in the pre-mRNA splicing pathway to facilitate spliceosome disassembly. | Increase 2.1 |
| U30602 | **Adenylate cyclase 9 (Adcy9);** cAMP biosynthesis; represents a "general" functional G protein-regulated adenylyl cyclase found in brain and In most somatic tissues; may play a fundamental role in situations where fine interplay between intracellular ca2+ and cAMP determines the cellular function; may be a physiologically relevant docking site for calcineurin | Increase 2.3 |
| D87903 | **ADP-ribosylation factor 6 (Arf6);** a family of small guanine nucleotide binding proteins that act as cofactors for ADP-ribosylation; involved in the formation of transport vesicles, facilitating the action of transport proteins | Increase 3.6 |
| X13605 | **H3 histons, family 3B (H3f3b);** chromosome organization and biogenesis | Increase 2.1 |
| X51438 | **Vimentin (vim);** intermediate filament protein | increase 2.9 |

**TABLE 5**

| GROUPS OF GENES WHICH DECREASED EXPRESSION WITH ST-CR (more than 1.8 fold). AGING AND LT-CR HAD NO EFFECT ON THE EXPRESSION OF THESE GENES. | | |
|---|---|---|
| GENE- BANK # | GENE | ST-CR |
| | STRESS RESPONSE/DETOXIFICATION | |
| U70475 | **Nuclear, factor, erythroid derived 2, like 2 (Nfe212);** transcription regulation; essential for the transcriptional induction of phase II enzymes and the presence of a coordinate transcriptional regulatory mechanism for phase II enzyme genes; The induction of phase II detoxifying enzymes is an important defense mechanism against intake of xenobiotics | Decrease -2.1 |
| J03549 | **Cytochrome P450, 2a4 (Cyp2a4);** steroid 15 alpha-hydroxylase; expression of Cyp2a4 is a female characteristic in liver microsomes, whereas higher levels of activity are found in male than in female kidney; androgen regulates expression | Decrease -2.2 |
| M21855 | **Cytochrome P450, 2b9, phenobarbital inducible, type a (Cyp2b9);** | Decrease -2.4 |
| X60452 | **Cytochrome P450, steroid inducible 3a11 (Cyp3a11);** monooxygenase, catalyzes erythromycin N- demethylation, nifedipine oxidation, and testosterone 6β-hydroxylation; hepatic levels of Cyp3a11 mRNA are greatly increased by treatment with dexamethasone | Decrease -3.3 |

| | METABOLISM / CATABOLISM | |
|---|---|---|
| U49878 | **3-hydroxy-3-methylglutaryl-Coenzyme A lyase (Hmgcl);** a mitochondrial matrix enzyme that catalyzes the cleavage of 3-hydroxy-3-methylglutaryl CoA to acetoacetic acid and acetyl *Co*A, the final reaction of both ketogenesis and leucine catabolism; Mitochondrial P-oxidation of long-chain fatty acids (LCFA) is essential for mammalian life. Regulation of LCFA catabolism influences cell signal pathways and apoptosis, as well as producing energy | Decrease -2.3 |
| Z14050 | **Dodecenoyl-Coenzyme A delta isomerase (3,2 trans-enoyl-Coenyme A isomerase) (Dci);** fatty acid metabolism; a key enzyme of mitochondrial beta-oxidation of unsaturated fatty acid | Decrease -2.5 |
| AF030343 | Enoyl coenzyme A hydratase 1, peroxisomal (Ech1); fatty acid metabolism | Decrease -2.7 |
| X58426 | **Hepatic lipase (Hpl);** catalyzes hydrolysis of triglycerides and phospholipids in hepatic tissue, where it is localized primarily on sinusoidal surfaces; an Important enzyme In HDL metabolism; | Decrease -2.7 |
| X02520 | **Lactate dehydrogenase 1, A chain (Ldh1);** catalyzes the interconversion of lactate and pyruvate; metabolism, . final step in anaerobic glycolysis | Decrease -2.3 |
| AJ001118 | **Monoglyceride lipase (Mgll);** catalyzes the last step in the hydrolysis of stored triglycerides in the adipocyte and presumably also complements the action of lipoprotein lipase in degrading tri-glycerides from chylomicrons and very low density lipoproteins | Decrease -3.0 |
| M93275 | **Adipose dlfferentiation related protein (Adfp);** expressed during differentiation of adipocytes; membrane-associated and adipose tissue specific in vivo; function unknown, may be involved in development and maintenance of adipose tissue; a fatty acid binding protein that specifically facilitates the uptake of long-chain fatty acids, may have a functional role in lipid metabolism | Decrease -3.2 |
| X64414 | **Low density lipoprotein receptor (Ldlr);** Cellular uptake of LDL is mediated by the LDL receptor. Ldlr plays an important role in cholesterol homeostasis, providing a source of cholesterol for membrane biogenesis and synthesis of bile acids and steroids, and on the other hand removing LDL from the circulation; | Decrease -2.2 |
| M88694 | **Thioether S-methyltransferase (Temt);** an important enzyme in the metabolism of sulfur and selenium-containing compounds in animals; catalyzes transfer of the methyl group from S-adenosylmethionine to X in compounds of the structure R-X-R', where X may be sulfur, selenium, or tellurium, and R and R' may be various organic groups; | Decrease -3.4 |

| | INTRACELLULAR SIGNALING | |
|---|---|---|
| M57696 | **Lyn A protein tyrosine kinase (IynA);** signal transduction; hematopoietic cell kinase; cell cycle regulator | Decrease -2.0 |
| X78682 | **Prohibitin (Phb);** antiproliferative activity; blocks entry into the S phase of the cell cycle | Decrease -2.3 |
| U59418 | **Protein phosphatase 2, regulatory subunit B (B56), gamma isoform (Ppp2r5c);** plays central roles in development, cell growth and transformation | Decrease -2.0 |
| U08110 | **RAN GTPase activating protein 1 (Rangap1);** a determinant of spindle assembly; important in development beginning immediately after conception | Decrease -3.4 |

| | TRANSCRIPTIONAL REGULATION | |
|---|---|---|
| M69293 | **Inhibitor of DNA binding 2 (ldb2);** ID (inhibitor of DNA binding) HLH proteins lack a basic DNA-binding domain but are able to form heterodimers with other HLH proteins, thereby inhibiting DNA binding; ID-2 may be an **Inhibitor of tissue-specific gene expression;** essential role in the generation of peripheral lymphoid organs and NK cells; control of cell differentiation | Decrease -2.0 |
| M60523 | **Inhibitor of DNA binding 3 (IDB3);** transcriptional activator; plays important role in cell differentiation | Decrease -3.4 |
| X62940 | **Transforming growth factor beta 1 Induced transcript 4 (Tgfb1i4);** transcription regulation; act on the promoter of the C-type natriuretic peptide (CNP), which is produced by most of the major endocrine glands and might be a local neuroendocrine regulator | Decrease -2.1 |

| | ION CHANNEL / TRANSPORTER | |
|---|---|---|
| X84215 | **Gap junction membrane channel protein beta 1 (GJb1);** gap junction proteins; contain ion exchange channels that generate signals throughout the tissue; Glucose mobilization from liver glycogen stores, after electrical stimulation of sympathetic nerves, is reduced by nearly 80% in Gjb1 null mice | Decrease -3.0 |
| X82438 | **Solute carrier family 16 (monocarboxylic acid transporters), member 1 (SIc16a1);** cell plasma membrane transporter; lactate transport; Transport of lactic acid, either outward as an end product of glycolysis or inward to serve as a substrate for respiration or gluconeogenesis, is required by most cells | Decrease -2.0 |

| | OTHERS | |
|---|---|---|
| D14883 | **CD82 antigen (Cd82);** belongs to a member of the transmembrane 4 superfamily; associates with CD4 or CD8 and delivers costimulatory signals for the TCR/CD3 pathway; a dominant epitope recognized by most of the mAb; an activation/differentiation marker of mononuclear cells; highly N-glycosylated with different patterns on various cells; identical to KAl1, a tumor metastasis suppressor which is capable of inhibiting the processes of tumor metastasis without affecting tumorigenicity per se; it is likely to function as a cellular adhesion molecule and may be involved in the invasion step of tumor metastasis | Decrease -2.7 |
| AF030635 | **FK506 binding protein 8 (38 kDa) (Fkbp8);** not much is known, 95% homologous to the human immunophilin homolog huFKBPr38, one of a family of proteins that are thought to interface with a wide range of Intracellular signal transduction systems; differentially expressed in Schwannoma cells and central nervous system neurons in vivo | Decrease -2.1 |
| X65582 | **Procollagen, type VI, alpha 2 (Col6a2);** cell adhesion | Decrease -2.0 |
| M55154 | **Transglutaminase 2, C polypeptide (Tgm2);** catalyzes the cross-linking posttranslational modification of proteins by transamidation of glutamine residues and the conjugation of polyamines to proteins; can be induced in mouse peritoneal macrophages by retinoic acid or by the active form of vitamin D, 1α,25-dihydroxyvitamin D3 | Decrease -3.3 |
| M95200 | **Vascular endothelial growth factor (Vegf);** growth factor active in angiogenesis, and endothelial cell growth; induces endothelial proliferation and vascular permeability | Decrease -1.9 |

### 6. LT-CR exclusively altered the expression of 30 genes at either young or old age, but not at both ages.

There were 30 genes that responded to CR in either young or old mice, but not in both age groups. For these genes, LT-CR increased the expression of 19 (Table 6), and decreased the expression of 11 (Table 7).
This group of genes is unique and interesting because it is the only group in which LT-CR induced changes in expression (increases or decreases) in young mice were not observed in old mice. These effects of CR are in contrast to those observed for the other 3 groups of genes discussed above (Figs. 8-10). In these groups, the effect of LT-CR observed in young mice were always found in the old mice.

ST-CR reproduced similar effects on 7 of 12 genes (58%) of the genes which changed expression in old LT-CR mice. Of these, 6 of 8 genes (75%) increased expression (Table.6) and 1 of 4 (25%) decreased expression (Table 7). Thus, ST-CR also reproduced the majority of these changes in old mice.

### OVERALL CONCLUSION

Overall, only 2- to 4-weeks of ST-CR reproduced nearly 65% of the changes induced by LT-CR in old mice. These results strongly indicate that ST-CR can be used to rapidly evaluate the effects of drugs and treatments on the majority of the gene expression changes induced by LT-CR.

**TABLE 6**

| GENES WHICH INCREASED EXPRESSION WITH LT-CR (more than 1.8 fold) EITHER IN YOUNG (EARLY INCREASE) OR OLD MICE (LATE INCREASE) ONLY. | | | |
|---|---|---|---|
| **GENE-BANK #** | **GENE** | **LT-CR** | **ST-CR** |
| | **STRESS RESPONSE / DETOXIFICATION** | | |
| | | | |
| U48420 | **Glutathione S-transferase, theta 2 (Gstt2);** Glutathione S-transferases (GSTs) are phase II metabolizing enzymes which catalyze the conjugation of glutathione (GSH) to electrophilic substrates and possess selenium-independent glutathione peroxidase activity; an important part of the cellular detoxification system and, perhaps, evolved to protect cells against reactive oxygen metabolites. Theta is considered the most ancient among the GSTs and theta-like GSTs are found in mammals. | Early increase 2.0 | Increase 2.2 |
| K02236 | **Metallothionein 2 (Mt2);** small protein capable of binding metal atoms, may play an important role in heavy metal detoxification and metabolism; a 17-base pair metal-regulatory element (MRE) has been implicated in the response of Mt1 and Mt2 to heavy metals; mutants with increased resistance to cadmium were shown to have increased expression of metallothionein due either to amplification or to increased transcription of Mt1 and Mt2 | Early Increase 2.8 | increase 6.3 |
| U35456 | **Glutathione synthetase (Gss);** catalyzes the last step in the synthesis of glutathione which has a variety of significant biochemical functions such as protection against oxidative damage, maintenance of thiol groups of proteins and of low molecular compounds, and participation in amino acid transport; malfunctioning of GS results in disorders including metabolic acidosis, 5-oxoprolinuria, neurological dysfunction, haemolytic anaemia and in some cases is probably lethal | Early increase 2.0 | No Effect |
| U96746 | **Peroxiredoxin 4 (Prdx4);** also known as antioxidant enzyme AOE372; a recently cloned fourth member of peroxiredoxin, a thioredoxin peroxidase; plays a role in protecting protein free thiol groups against oxidative damage and thioredoxin-dependent peroxidase activity; suggested to be an immediate regulator of H2O2-mediated activation of NF-kB; expressed ubiquitously; Prdx4 has been reported to prevent ROS production stimulated by expression of P53, thereby inhibiting P53-induced apoptosis; Thus, Prdx4 is critically involved in intracellular redox signaling. Peroxiredoxins are evolutionarily conserved in all organisms. The ubiquity and structural conservation of peroxiredoxins suggest that they serve fundamentally important functions | Late Increase 2.5 | No Effect |
| U90535 | **Flavin containing monooxygenase 5 (Fmo5);** phase I drug-metabolizing system in the liver; At least five FMO forms have been reported in mammals. Male rat liver contains FMO1 as a major form and FMO3 as a minor one. FMOs are now thought to be involved in the metabolism of a variety of structurally diverse xenobiotics, as well as the P450 system | Late Increase 2.1 | Increase 3.8 |

| | METABOLISM /BIOSYNTHESIS | | |
|---|---|---|---|
| M74495 | **Adenylosuccinate synthetase 1, muscle (Adss1);** synthesis of adenylosuccinate from inosine monophosphate and aspartate is carried out by the enzyme adenylosuccinate synthetase, as the first step in the formation of adenine nucleotides from inosine monophosphate; not listed in liver; an acidic isoform which occurs in non-muscle tissues is encoded by a separate gene, Adss2 | Late Increase 2.3 | Increase 2.6 |
| L32836 | **S-adenosylhomocystelne hydrolase (Ahcy);** arises from reactions transferring methyl groups from S-adenosyl methionine, an important source for nucleic acid methylation. | Late Increase 2.5 | Increase 2.6 |
| U00445 | **Glucose-6-phosphatase, catalytic (G6pc);** the key enzyme in homeostatic regulation of blood glucose levels, catalyzing the terminal step in gluconeogenesis and in glycogenolysis. | Late Increase 2.0 | No Effect |

| | TRANSPORTERS | | |
|---|---|---|---|
| U27316 | **Solute carrier family 25 (mitochondrial carrier; adenine nucleotide translocator), member 5 (Slc25a5);** mitochondrial inner membranes contain various proteins that transport metabolites. Adenine nucleotide translocator protein carries ATP from the mitochondrial matrix into the intermembrane space and the ADP in the reverse direction. | Late Increase 2.0 | Increase 2.0 |
| X61433 | **ATPase, Na+/K+ transporting, beta 1 polypeptide (Atp1b1);** the non-catalytic component of the active enzyme, which catalyzes the hydrolysis of ATP coupled with the exchange of Na and K ions across the plasma membrane; the beta subunit regulates, through assembly of alpha/beta heterodimers, the number of sodium pumps transported to the plasma membrane; | Early Increase 4.7 | No Effect |

| | RIBOSOMAL PROTEIN | | |
|---|---|---|---|
| U12403 | **Ribosomal protein L10A (Rpl10a);** belongs to the L1p family of ribosomal proteins; down-regulated in the brain and liver during mouse development; also down-regulated in the thymus by cyclosporin-A (CsA) | Early Increase 2.1 | No Effect |
| L04280 | **Ribosomal protein L12 (Rpl12);** structural protein of ribosome; binds directly to 26s ribosomal RNA | Early Increase 2.0 | No Effect |

| | OTHER | | |
|---|---|---|---|
| U41736 | **Ancient ubiquitous protein (Aup1);** encodes a protein of 410 aa; ubiquitously expressed in mouse tissue; has an evolutionary relationship to the C. elegans predicted protein F44b9.5 is indicated by the 35% identity and 53% conservation of the amino acid sequence | Early Increase 2.1 | No Effect |
| M59821 | **Immediate early response 2 (ler2);** serum growth factors or tumor promoting agents inducible; transcriptional activation of this gene is mediated by a promoter fragment upstream of the transcription start site; the fragment contains binding sites for Ets family transcription factors and for the serum response factor | Early increase 2.0 | No Effect |
| M25244 | **Lysosomal membrane glycoprotein 1(Lamp1);** highly glycosylated protein distributed within the cell primarily in the lysosome; under certain circumstances, e.g. after platelet activation, during granulocytic differentiation and activation, and on cytotoxic T lymphocytes it is also found at the cell surface; cell surface-expressed LAMP-1 presents carbohydrate ligands to selections, also implicated in tumor cell metastasis | Early Increase 2.0 | No Effect |
| U16740 | **Capping protein alpha 1. (Cappa1);** F-actin capping proteins, heterodimers of alpha (vertebrates have three alpha Isoforms: alpha. 1, alpha 2, alpha 3) and beta subunits, bind in a Ca2+-independent manner to the fast growing ends of actin filaments (barbed end) thereby blocking the exchange of subunits at these ends; unlike other capping proteins (such as gelsolin and severin), these proteins do not sever actin filaments | Early Increase 2.5 | No Effect |
| L47599 | **Troponin T2, cardiac (Tnnt2);** troponin T is the tropomyosin-binding subunit of troponin, the thin filament regulatory complex which confers calcium-sensitivity to striated muscle actomyosin ATPase activity | Early Increase' 2.6 | No Effect |
| U96116 | **Hydroxyacyl-Coenzyme A dehydrogenase, type II (Hadh2);** has 100% sequence homology (1000 bp coding region) to amyloid beta-peptide binding protein (ERAB); the localization of ERAB to the ER and mitochondria suggests a complex interaction with components of the programmed cell death machinery. The interaction of A beta with ERAB further links oxidoreductase activity with both apoptosis and amyloid toxicity. | Late Increase 2.5 | Increase 2.7 |
| Z30970 | **Tissue Inhibitor of metalloproteinase 3 (Timp3);** involved in gradation of collagen fibers, the key units of connective tissue architecture, is initiated by proteinase enzymes such as the matrix metalloproteinase collagenase. A specific inhibitor of collagenase activity, Timp combines with the enzyme to form an inactive complex. It has been suggested that the ubiquitous inhibitor is normally in excess, and that pathological conditions in which collagen breakdown occurs are associated with deficiencies of TIMP. The extracellular matrix is increasingly implicated in the pathology of degenerative and/or neoplastic diseases. For example, TIMP proteins, which normally inactivate matrix met-alloproteinases involved in degrading the structural components of the extracellular matrix (e.g. collagens, fibronectin, and gelatins), were found to prevent cell migration and metastasis) depending on the experimental model, TIMP proteins can either promote or inhibit oncogenic transformation, suggesting that their function is cell type-specific. Timp3 is downstream targets of c-RAS within the EGF/EGFR signal transduction pathway, it has been shown to inhibit oncogenic transformation and is specifically repressed in EGF-transformed cells. | Late Increase 1.8 | Increase 3.2 |

**TABLE 7**

| GENES WHICH DECREASED EXPRESSION WITH LT-CR (more than 1.8 fold) EITHER IN YOUNG (EARLY DECREASE) OR OLD MICE (LATE DECREASE) ONLY. | | | |
|---|---|---|---|
| GENE- BANK # | GENE | LT-CR | ST-CR |
| | MOLECULAR CHAPERONE | | |
| X17069 | **FK506 binding protein 4 (59 kDa) (Fkbp4);** FK506 binding proteins are a subfamily of immunophilins, which bind FK506 and rapamycin; displays peptidyl-prolyl isomerase (rotamase) activity, which presumably facilitates the folding and trafficking of proteins, though rotamase inhibition after binding of the corresponding immunosuppressant ligand is not responsible for the immunosuppressive effects of the drugs, also interact with diverse proteins involved in different signal transduction pathways | Early Decrease -2.5 | Decrease -3.1 |

| | CELL GROWTH /INTRACELLUAR SIGNALING | | |
|---|---|---|---|
| X95280 | **G0/G1 switch gene 2 (G0s2);** cell cycle regulation; potential oncogene and regulator of latent HIV; demonstrated to be predominantly expressed in adipose tissue during late murine embryonic development | Early Decrease -3.3 | No Effect |
| M31885 | **Inhibitor of DNA binding 1 (Idb1);** has the helix-loop-helix motif, but lack the basic region. Thus they can form heterodimers with other bHLH proteins, but cannot bind DNA, and may provide negative regulation for these other proteins by interfering with their ability to bind DNA | Early Decrease -2.1 | No Effect |
| U92068 | **RAD51 like 1 (S. cerevisiae) (Rad51I1);** genes from the recA/RAD51 family play essential roles in homologous recombination in all organisms; functions in homologous recombination-repair pathways | Early Decrease -2.1 | No Effect |
| U72881 | **Regulator of G-proteln signaling 16(Rgs16);** GRS proteins modulate G protein-mediated signaling pathways by acting as GTPase-activating proteins for Gi, Gq, and G12 alpha-subunits of heterotrimeric G proteins | Early Decrease -1.9 | No Effect |

| | IMMUNE RESPONSE | | |
|---|---|---|---|
| U60438 | **Serum amyloid A 2 (Saa2);** SAA family comprises a number of differentially expressed apolipoproteins, acute-phase SAAs (A-SAAs) and constitutive SAAs (C-SAAs). A-SAAs are major acute-phase reactants, liver is the primary site of synthesis of both types; Saa2 is a A-SAA; A-SAA is potentially involved in the pathogenesis of several chronic inflammatory diseases: it is the precursor of the amyloid A protein deposited in amyloid A amyloidosis, and it has also been implicated in the pathogenesis of atherosclerosis and rheumatoid arthritis. | Early Decrease -2.2 | No Effect |
| M29008 | **Mouse complement factor H-related protein mRNA:** one of the four complement factor H-related transcripts cloned from mouse liver. -2.0 | Early Decrease | No Effect |
| L32974 | **Interferon-induced protein with tetratricopeptide repeats 3 (Ifit3);** immune response, function unknown; the induction may occur via a PKC pathway | Late Decrease -2.1 | No Effect |
| AF027865 | **Major Histocompatibility Locus class II region, contains ATP-binding cassette, sub-family B (MDR/TAP), member 2 (Abcb2),** histocompatibility 2, class II antigen A, beta 1 (H2-Ab1) and other genes. The Abcb2 and Abcb3 genes encode a heterodimeric protein complex located on the membrane of the ER, and responsible for the transport of endogenous peptides out of the cytosol into the ER lumen; they are believed to be involved in generation and transport of endogenous peptides for class I antigen processing. | Late Decrease -3.8 | No Effect |
| U19482 | **Small Inducible cytokine A9 (Scya9);** member of C-C chemokine family; may play a role in inflammatory processes; expressed constitutively by a wide variety of tissues | Late Decrease -2.0 | No Effect |

| | OTHERS | | |
|---|---|---|---|
| Z11997 | **High mobility group protein 1 (Hmg1);** one of the most abundant non-histone chromosomal proteins in the nuclei of higher eukaryotes; considered as a structural protein of chromatin; critical for proper transcriptional control by specific transcription factors; in most cases, it is stimulatory for transcription | Late Decrease -2.0 | Decrease -2.9 |

### Example 17

### Gene expression in STZ-diabetic mice

Streptozotocin (STZ) induces diabetes. Mice receiving three treatments with STZ were diabetic for about 4 weeks. Diabetes reduces insulin levels to almost zero. CR has a similar effect in that it lowers insulin levels, although not as low as in STZ-treated animals. Also, while CR lengthens life span, STZ has the opposite effect and shortens life span.

Figure 11 shows pairwise comparison of global gene expression correlation coefficients for each possible mouse pair. The results indicate that hepatic gene expression is very different between young CR, young control and STZ-diabetic mice. In conclusion, lowering insulin in the pathological way found in serious diabetes is insufficient to produce the gene expression profile or the life-span effects observed with CR.

### Example 18

### Gene expression in aminoguanidine treated mice

Aminoguanidine is believed to retard aging by preventing cross-linking of protein initiated by the aldehyde form of glucose. However, mice fed aminoguanidine exhibited little or no effect on life span. However, a large effect on gene expression was observed (Figure 12). Gene expression for aminoguanidine-treated mice did not correlate with either old CR or old control. In conclusion, although aminoguanidine has little effect on aging in mice, major differences in gene expression are observed. These effects are not like those of CR, and this is consistent with the absence of a strong effect on the life-span of mice.

### Example 19

To determine whether certain interventions mimic calorie restriction in mice, the following groups of mice are prepared.
Group 1: Controls
Group 2: Troglitazone (synthetic proposed calorie restriction mimetic drug that lowers insulin levels in rats and mice, lowers blood pressure and triglycerides, inhibits free radicals, increases mitochondrial mass, and doesn't seem to change food intake in rodents): treatment starts at 10 months
Group 3: IGF-1 (natural proposed calorie restriction mimetic hormone that lowers both insulin and glucose levels and which may be directly involved in the basic mechanisms of aging; has rejuvenating effects on immune, muscular, and other systems): treatment starts at 12 months
Group 4: ALT-711 (or other AGE breaking agent: proposed calorie restriction mimetic that acts by reversing the effects of elevated glucose levels as they occur or after they occur, rather than by reducing glucose levels): treatment starts at 18 months.

Animals in all groups will receive the same, known amount of food throughout the study.

Troglitazone and IGF-1 doses will be chosen to set glucose and insulin levels in the range for young or preferably calorie-restricted animals. Glucose and insulin will be measured but not controlled in the control and ALT-711 groups Troglitazone will be supplied at a dose of 0.2% of the diet (standard for troglitazone studies for other purposes). Similarly, ALT-71 will be incorporated into the diet. A low (non-toxic) level of ALT-711 is used that will remain constant over time.

It is assumed that IGF-1 will be'supplied by injection (3 times per week, minimum) unless a continuous delivery method can be arranged. The preferred dosage method is implantation of non-dividing IGF-1-secreting cells, to attain steady IGF-1 levels, and if possible, this will be done. If this is not possible, IGF-1 will be obtained as a gift from Genentech or another manufacturer. Other possible alternatives to injection are: osmotic minipump; injection of IGF-1 into subcutaneous slow-release reservoirs; infusion by means of minipumps used by Celtrix; use of skin patches that allow slow-release to the body.

There will be 60 animals in each longevity-testing group (LTG). Each LTG will be accompanied by another set of, on average, 40 similarly-treated animals, which will be set aside for sacrifice to permit biochemical assays and histological documentation of the condition of the animals at fixed ages (sacrifice group, SG). In the case of the IGF-1 and troglitazone groups, some animals will be earmarked for pilot dose-finding experiments in a manner that will allow the average SG size to remain at 40, as described below. The groups earmarked for dose-verification will be referred to as the pilot dose groups, or POGs.

For troglitazone, about a 2-month supply of each of three troglitazone diets (containing 0.1%, 0.2%, or 0.3% troglitazone) will be initially ordered. The main 0.2% troglitazone dose will be tested on a small pilot mouse population before committing the troglitazone group proper to this dose. It 0.2% troglitazone is not found to yield the expected changes in circulating insulin after 2 weeks on the 0.2% troglitazone, the diet will be changed to the more appropriate dose diet at that time and verified on a second small pilot mouse population.

Similarly, some animals will be used for IGF-1 injection pilot experiments to determine the proper starting dose.

At age 12 months: Sacrifice 3 animals/SG to obtain common baseline group of 12 animals to be compared to all subsequent results. This is the middle-aged universal control group. All subsequent data can be compared to the results for this pooled group.

At age 12.5 months: Begin the IGF-1 PDG with 7 mice given the best estimated dose of IGF-1. Sacrifice two weeks later for determination of insulin and glucose levels. Begin a verification/second trial dose of IGF-1 at 13 months, 1 week of age, and sacrifice this second PDG at 13 months, 3 weeks of age. Assuming the assays for insulin and glucose can be completed in 1 week, this regimen will allow the final dose for the LTG to be determined prior to age 14 months. Similarly, at 12.5 months, place 7 mice on the 0.2% troglitazone diet. Two weeks later, sacrifice and assay for insulin and glucose. Begin adjusted-dose or verification dose group at 13 months, 1 week and sacrifice after two weeks.

At age 14 months: Begin troglitazone and IGF-I at the experimentally-determined or estimated optimal doses for each.

At age 15 months: Sacrifice six animals from the IGF-1 and troglitazone SGs for determinations of glucose, insulin, and all other endpoints involved in the study. If necessary, adjust the IGF-1 dose again (both in the LTG and the untapped portion of the IGF-1 SG) and/or order diet with a modified troglitazone content. Sacrifice three animals each from the SGs for the controls and the ALT-11 groups and pool to create a common group of six animals for comparison to the IGF-1 and troglitazone groups.

At age 18 months: same as at 15 months, but use 7 mice/SG for IGF-1 and troglitazone and 4 mice/SG for the control and for the ALT-711 group. Begin the ALT-711 groups on ALT-711 immediately after this sampling.

At around 27 months (⁻24-30 months): Sample all remaining surviving SG mice.

If the total initial numbers of mice in the sacrifice groups for treatments 1, 2, 3, and 4 are 30, 50, 50, and 30, respectively, then if there were no mortality in any of these groups, there would be 20 animals left in each SG at the time of final sampling. But if we assume that only 1/3 of this number will be alive, then about 7 animals will remain to be sampled at the final sample time, or about the minimum required for statistical significance. If the mean survival rate at 27 month is over 73%, the 27 month end point may be postponed to a greater age.

In addition to other biochemical markers, assays may include:
heart and thymus volume and histology;
autoantibody titer;
T and B cell characteristics;
protein or albumin concentration in bladder urine at sacrifice;
molecular glycation indices;
protein carbonyl content or other free radical/oxidation indices; and
incidence of neoplasia, esp. of prostate and breast.

## Claims

1. A method of identifying an intervention that mimics the effects of caloric restriction in cells, comprising:
exposing a biological sample comprising cells in vitro or a non-human mammal to an intervention;
waiting a specified period of time;
assessing changes in gene expression levels, levels of RNA, protein, or protein activity levels related to one or more biomarkers of aging; and
identifying said intervention as one that mimics the effects of caloric restriction if one or more changes in said levels also occurs in short term caloric restriction.

2. The method of claim 1, wherein the short term caloric restriction is in a reference animal for two to six weeks.

3. The method of claim 1, wherein the short term caloric restriction is in a reference animal for less than four weeks.

4. The method of claim 1, wherein the short term caloric restriction is in a reference animal for less than two weeks.

5. The method of claim 1, wherein said biological sample comprises cells.

6. The method of claim 5, wherein said cells are obtained from a mammal.

7. The method of claim 6, wherein said mammal is a mouse.

8. The method of claim 1, wherein the biomarker of aging is a chaperone protein.

9. The method of claim 8, wherein said chaperone protein is GRP78.

10. The method of claim 1, wherein said biomarker is a protein involved in apoptosis.

11. The method of claim 1, wherein the specified period of time is six weeks or less.

12. The method of claim 11, wherein the specified period of time is four weeks or less.

13. The method of claim 12, wherein the specified period of time is two weeks or less.

14. The method of claim 13, wherein the specified period of time is two days or less.

15. A method according to claim 1, wherein changes in gene expression are evaluated using an oligvnucleotide-based high density array.

16. The method of claim 15, wherein the array contains genes for stress proteins.

17. The method of claim 15, wherein the array contains genes associated with inflammation response.

18. The method of claim 15, wherein the array contains genes associated with apoptosis.

19. The method of claim 15, wherein the array contains genes selected from the group consisting of transcription factor proteins, transmembrane channel proteins and transporter proteins.

20. The method of claim 1, wherein a non-human animal is used.

21. The method of claim 20, additionally comprising determining changes in said levels in a reference animal, wherein the reference animal has been on a short term calorie restricted diet and wherein said changes are used in the identifying step to identify the intervention as one that mimics the effects of calorie restriction.

22. The method of claim 21, wherein the reference animal has been on a calorie restricted diet for less than about 4 weeks.

23. The method of claim 21, wherein the reference animal has been on a calorie restricted diet for less than about 2 weeks.

24. The method of claim 20, wherein said test animal is a mouse.

25. The method of claim 20, wherein changes in gene expression are assessed in said test animal.

26. The method of claim 20 which further comprises:
obtaining a gene expression profile from a calorie-restricted reference animal;
comparing changes in gene expression for the test animal to the gene expression profile of the calorie-restricted reference animal; and
identifying said intervention as one that mimics the effects of calorie restriction if the gene expression profile of the test animal is statistically similar to the gene expression profile of the calorie restricted animal.

27. The method of claim 26, wherein the gene expression profile of the test animal is determined to be statistically similar to the gene expression of the calorie restricted animal by one-way ANOVA followed by Fisher's test (*P*<0.05).

## Patentansprüche

1. Verfahren zum Identifizieren einer Intervention, welche die Wirkungen einer kalorischen Beschränkung in Zellen nachahmt, umfassend:
Exponieren einer Zellen umfassenden biologischen Probe in vitro oder eines nichthumanen Säugers an eine Intervention;
Warten für einen vorgegebenen Zeitraum;
Bewerten von Veränderungen in den Gen-Expressionsspiegeln, Spiegeln von RNA, Protein- oder Proteinaktivitätsspiegeln in Bezug auf einen oder mehrere Biomarker für das Altem; und
Identifizieren der Intervention als einer, welche die Wirkungen der kalorischen Beschränkung nachahmt, wenn eine oder mehrere Veränderungen in den Spiegeln auch bei einer kurzzeitigen kalorischen Beschränkung auftreten.

2. Verfahren gemäß Anspruch 1, wobei die kurzzeitige kalorische Beschränkung bei einem Referenztier während zwei bis sechs Wochen vorgenommen wird.

3. Verfahren gemäß Anspruch 1, wobei die kurzzeitige kalorische Beschränkung bei einem Referenztier für weniger als vier Wochen vorgenommen wird.

4. Verfahren gemäß Anspruch 1, wobei die kurzzeitige kalorische Beschränkung bei einem Referenztier für weniger als zwei Wochen vorgenommen wird.

5. Verfahren gemäß Anspruch 1, wobei die biologische Probe Zellen umfasst.

6. Verfahren gemäß Anspruch 5, wobei die Zellen von einem Säuger erhalten werden.

7. Verfahren gemäß Anspruch 6, wobei der Säuger eine Maus ist.

8. Verfahren gemäß Anspruch 1, wobei der Biomarker für das Altem ein Chaperonprotein ist.

9. Verfahren gemäß Anspruch 8, wobei das Chaperonprotein GRP78 ist.

10. Verfahren gemäß Anspruch 1, wobei der Biomarker ein an der Apoptose beteiligtes Protein ist.

11. Verfahren gemäß Anspruch 1, wobei der vorgegebene Zeitraum sechs Wochen oder weniger beträgt.

12. Verfahren gemäß Anspruch 11, wobei der vorgegebene Zeitraum vier Wochen oder weniger beträgt.

13. Verfahren gemäß Anspruch 12, wobei der vorgegebene Zeitraum zwei Wochen oder weniger beträgt.

14. Verfahren gemäß Anspruch 13, wobei der vorgegebene Zeitraum zwei Tage oder weniger beträgt.

15. Verfahren gemäß Anspruch 1, wobei die Veränderungen in der Genexpression unter Verwendung eines hochdichten Arrays auf Oligonukleotidbasis evaluiert werden.

16. Verfahren gemäß Anspruch 15, wobei das Array Gene für Stressproteine enthält.

17. Verfahren gemäß Anspruch 15, wobei das Array mit einer Entzündungsantwortreaktion assoziierte Gene enthält.

18. Verfahren gemäß Anspruch 15, wobei das Array mit Apoptose assoziierte Gene enthält.

19. Verfahren gemäß Anspruch 15, wobei das Array Gene enthält, die aus der Gruppe, bestehend aus Transkriptionsfaktorproteinen, Transmembrankanalproteinen und Transporterproteinen, gewählt sind.

20. Verfahren gemäß Anspruch 1, wobei ein nicht-humanes Tier verwendet wird.

21. Verfahren gemäß Anspruch 20, zusätzlich umfassend das Bestimmen von Veränderungen der besagte Spiegel in einem Referenztier, wobei das Referenztier auf eine kurzzeitige Diät mit kalorischer Beschränkung gesetzt wurde und wobei die genannten Veränderungen in dem Identifizierungsschritt zur Identifizierung der Intervention als einer, welche die Wirkungen einer kalorischen Beschränkung nachahmt, genutzt werden.

22. Verfahren gemäß Anspruch 21, wobei das Referenztier weniger als etwa 4 Wochen lang auf eine Diät mit kalorischer Beschränkung gesetzt wurde.

23. Verfahren gemäß Anspruch 21, wobei das Referenztier weniger als etwa 2 Wochen lang auf eine Diät mit kalorischer Beschränkung gesetzt wurde.

24. Verfahren gemäß Anspruch 20, wobei das Versuchstier eine Maus ist.

25. Verfahren gemäß Anspruch 20, wobei Veränderungen in der Genexpression in dem Versuchstier bewertet werden.

26. Verfahren gemäß Anspruch 20, weiterhin umfassend:
Erhalten eines Genexpressionsprofils von einem Referenztier mit kalorischer Beschränkung;
Vergleichen von Veränderungen in der Genexpression für das Versuchstier mit dem Genexpressionsprofil des Referenztiers mit kalorischer Beschränkung; und
Identifizieren der Intervention als einer, welche die Wirkungen der kalorischen Beschränkung nachahmt, wenn das Genexpressionsprofil des Versuchstiers statistisch dem Genexpressionsprofil des Tiers mit kalorischer Beschränkung ähnelt.

27. Verfahren gemäß Anspruch 26, wobei das Genexpressionsprofil des Versuchstiers als statistisch ähnlich zu der Genexpression des Tiers mit kalorischer Beschränkung durch Ein-Weg-ANOVA, gefolgt von einem Fisher-Test (P < 0,05), bestimmt wird.

## Revendications

1. Procédé d'identification d'une intervention mimant les effets d'une restriction calorique chez des cellules, lequel procédé comporte les étapes suivantes :
- exposer à une intervention un échantillon biologique comprenant des cellules, *in vitro,* ou un mammifère non-humain ;
- attendre un laps de temps donné ;
- déterminer les variations des niveaux d'expression de gènes, des taux d'ARN, des taux de protéines ou des niveaux d'activité de protéines, en rapport avec un ou plusieurs biomarqueurs du vieillissement ;
- et identifier ladite intervention comme étant une intervention qui mime les effets d'une restriction calorique si une ou plusieurs variations desdits taux et niveaux se produisent aussi lors d'une restriction calorique à court terme.

2. Procédé conforme à la revendication 1, pour lequel la restriction calorique à court terme dure deux à six semaines chez un animal de référence.

3. Procédé conforme à la revendication 1, pour lequel la restriction calorique à court terme dure moins de quatre semaines chez un animal de référence.

4. Procédé conforme à la revendication 1, pour lequel la restriction calorique à court terme dure moins de deux semaines chez un animal de référence.

5. Procédé conforme à la revendication 1, dans lequel ledit échantillon biologique comprend des cellules.

6. Procédé conforme à la revendication 5, pour lequel lesdites cellules proviennent d'un mammifère.

7. Procédé conforme à la revendication 6, pour lequel ledit mammifère est une souris.

8. Procédé conforme à la revendication 1, dans lequel le biomarqueur du vieillissement est une protéine chaperon.

9. Procédé conforme à la revendication 8, dans lequel ladite protéine chaperon est la protéine GRP78.

10. Procédé conforme à la revendication 1, dans lequel ledit biomarqueur est une protéine impliquée dans l'apoptose.

11. Procédé conforme à la revendication 1, dans lequel le laps de temps donné est inférieur ou égal à six semaines.

12. Procédé conforme à la revendication 11, dans lequel le laps de temps donné est inférieur ou égal à quatre semaines.

13. Procédé conforme à la revendication 12, dans lequel le laps de temps donné est inférieur ou égal à deux semaines.

14. Procédé conforme à la revendication 13, dans lequel le laps de temps donné est inférieur ou égal à deux jours.

15. Procédé conforme à la revendication 1, dans lequel on évalue les variations de l'expression des gènes au moyen d'un réseau à haute densité à base d'oligonucléotides.

16. Procédé conforme à la revendication 15, dans lequel le réseau contient des gènes de protéines de stress.

17. Procédé conforme à la revendication 15, dans lequel le réseau contient des gènes associés à la réponse inflammatoire.

18. Procédé conforme à la revendication 15, dans lequel le réseau contient des gènes associés à l'apoptose.

19. Procédé conforme à la revendication 15, dans lequel le réseau contient des gènes choisis dans l'ensemble constitué par les gènes des protéines facteurs de transcription, les gènes des protéines canaux transmembranaires et les gènes des protéines transporteurs.

20. Procédé conforme à la revendication 1, dans lequel on emploie un animal non-humain.

21. Procédé conforme à la revendication 20, qui comporte en outre le fait de déterminer les variations desdits taux ou niveaux chez un animal de référence, lequel animal de référence a été mis à un régime de restriction calorique à court terme, et dans lequel procédé l'on se sert de ces variations, dans l'étape d'identification, pour identifier ladite intervention comme étant une intervention qui mime les effets d'une restriction calorique.

22. Procédé conforme à la revendication 21, dans lequel l'animal de référence a été soumis à un régime de restriction calorique durant moins d'environ quatre semaines.

23. Procédé conforme à la revendication 21, dans lequel l'animal de référence a été soumis à un régime de restriction calorique durant moins d'environ deux semaines.

24. Procédé conforme à la revendication 20, dans lequel ledit animal de test est une souris.

25. Procédé conforme à la revendication 20, dans lequel on détermine les variations de l'expression des gènes chez ledit animal de test.

26. Procédé conforme à la revendication 20, qui comporte en outre les étapes suivantes :
- déterminer un profil d'expression génétique chez un animal de référence soumis à une restriction calorique ;
- comparer les variations de l'expression des gènes chez l'animal de test à ce profil d'expression génétique chez un animal de référence soumis à une restriction calorique ;
- et identifier ladite intervention comme étant une intervention qui mime les effets d'une restriction calorique si le profil d'expression génétique observé chez l'animal de test est statistiquement similaire au profil d'expression génétique déterminé chez l'animal de référence soumis à une restriction calorique.

27. Procédé conforme à la revendication 26, dans lequel c'est en effectuant une analyse de la variance à une variable, suivie d'un test de Fisher (P < 0,05), que l'on détermine si le profil d'expression génétique observé chez l'animal de test est statistiquement similaire au profil d'expression génétique déterminé chez l'animal soumis à une restriction calorique.
